(19) Europäisches Patentamt
European Patent Office
Office européen des brevets

(11) EP 0 381 130 B1

(12) EUROPEAN PATENT SPECIFICATION

(45) Date of publication and mention of the grant of the patent:
**04.10.2001 Bulletin 2001/40**

(51) Int Cl.[7]: **C07D 213/36**, C07D 213/61, C07D 213/64, C07D 215/12, C07D 241/12, C07D 277/28, C07D 277/32, C07C 205/08

(21) Application number: **90101778.0**

(22) Date of filing: **30.01.1990**

(54) **Production of alpha-unsaturated amines**

Herstellung von alpha-ungesättigten Aminen

Préparation d'amines alpha-insaturées

(84) Designated Contracting States:
**CH DE FR GB IT LI**

(30) Priority: **31.01.1989 JP 2335689**

(43) Date of publication of application:
**08.08.1990 Bulletin 1990/32**

(73) Proprietor: **Takeda Chemical Industries, Ltd.**
**Chuo-ku, Osaka (JP)**

(72) Inventors:
- **Aoki, Isao**
**Kawanishi, Hyogo 666-01 (JP)**
- **Tabuchi, Takanori**
**Suita, Osaka 565 (JP)**
- **Minamida, Isao**
**Kawabe-gun, Hyogo 666-02 (JP)**

(74) Representative:
**von Kreisler, Alek, Dipl.-Chem. et al**
**Patentanwälte**
**von Kreisler-Selting-Werner**
**Postfach 10 22 41**
**50462 Köln (DE)**

(56) References cited:
**DE-A- 3 305 202**
**US-A- 2 928 883**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

BACKGROUND OF THE INVENTION

1. Field of the Invention

The present invention relates to a process for the production of α-unsaturated amines. More particularly, it relates to an improved process for the production of α-unsaturated amines useful as insecticides and their 1,1,1-trichloro-2-nitroethane intermediate.

2. Description of the Prior Arts

α-Unsaturated-amines of the formula (V) shown hereinbelow or their salts are excellent insecticidal compounds [cf. EP-302389A]. Further, the production of compounds of the formula

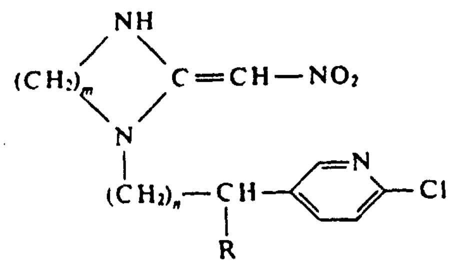

by reacting a diamine of the formula

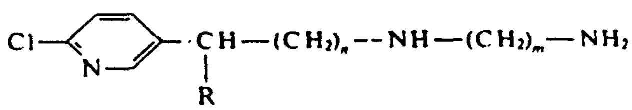

(in which formulae R is H or lower alkyl, m is 2, 3 or 4 and n is 0, 1, 2 or 3)
with a fluornitroethan derivative of the formula

$$CFX_1X_2\text{-}CH_2\text{-}NO_2$$

($X_1$ and $X_2$ being independently chlorine or fluorine) is known from DE-A-36 03 100.

The 1,1,1-trihalogeno-2-nitroethane intermediates can be prepared by the methods of the following formulas [1] and [2].

[1]

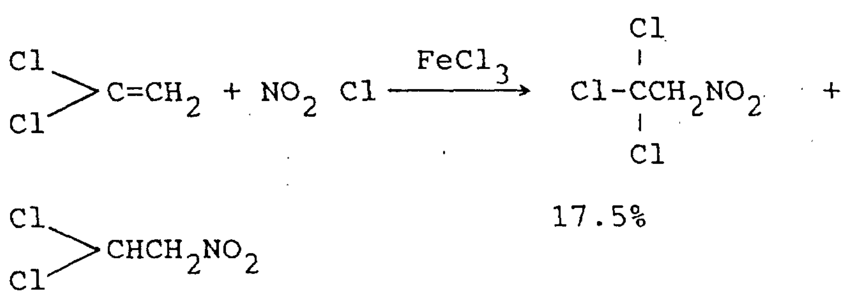


[cf. Izvest. Akad. Nauk S.S.S.R., Otdel. Khim. Nauk, 1958, 841 (C.A., 53, 1111i (1959))].

[2]

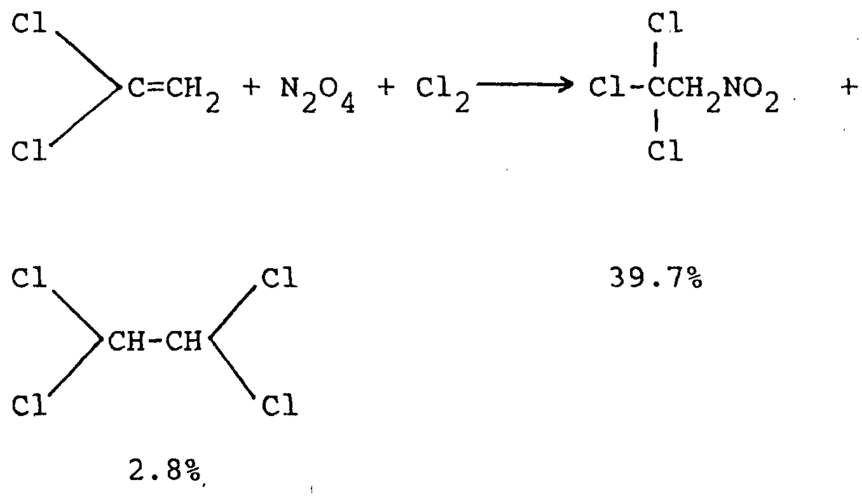


[cf. J. Org. Chem., 25, 1312 (1960)].

However, the method of the formula [1] mentioned above which reacts 1,1-dichloroethylene with nitryl chloride shows the disadvantages that nitryl chloride is explosive [cf. Ber. 75 B, 1323(1942)]; its generation must use fuming nitric acid and chlorosulfonic acid which are dangerous and difficult to handle; a large amount of waste sulfuric acid must be disposed and the yield of the object compound is low. The method of the formula [2] has many problems that very toxic dinitrogen tetroxide and chlorine gas must be used and the yield of the object compound is also low.

Also, the method of the formula [3] is known as the reaction of 1,1-dihalogenoethylene with nitric acid.

[3]

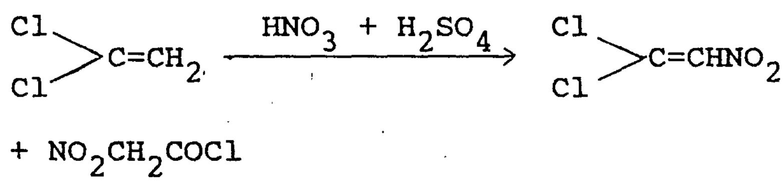


[cf. Probl. Organ. Sinteza. Akad. Nauk S.S.S.R., Otd. Obshchi. Tekhn. Khim. 1965. 60 (CA. 64, 8023b (1966))]. However, it is reported that the yield of this reaction is extremely low, i.e., 10.6% of 1,1-dichloro-2-nitroethylene and 37% of nitroacetyl chloride, respectively.

On the other hand, the methods of the formulas [4] and [5] are known to introduce nitro group and fluorine atom into 1,1-dihalogenoethylene (the latter is supposed to show reactivity entirely different from chlorine and bromine atoms).

[4]

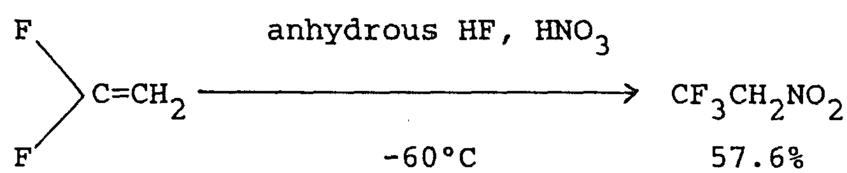


[Izv. Akad. Nauk SSSR, Ser. Kim., 1963, 1946(cf. CA. 60, 5325g)]

[5]

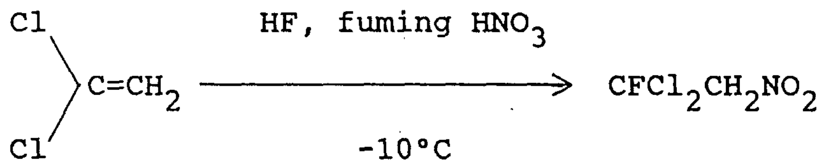


[Dokl. Akad. Nauk SSSR, 149, 330 - 333 (1963) (cf. CA. 59, 6215g)]

Also, improvements on the above methods [4] and [5] are disclosed in DE 3,305,201 and DE 3,305,202, which however do not afford 1,1,1-trichloro or tribromo-2-nitroethane as the object compound and further do not suggest or teach to react olefins with hydrogen chloride or hydrogen bromide and nitric acid to introduce chlorine or bromine atom as well as nitro group.

The reaction of halogenated olefins with hydrogen chloride or hydrogen bromide is required to use a catalyst such as a metal chloride or active carbon, because their reactivity is less due to reverse inductive effect (-I effect) of the halogen atom in the halogenated olefins. For instance, anhydrous ferric chloride is used in the reaction of 1,1-dichloroethylene with hydrogen chloride (cf. USP. 2,209,000).

On the other hand, the reactivity of hydrogen fluoride is different from other hydrogen halides. For instance, hydrogen fluoride is required to react with common olefins under pressure, although they tend to react with hydrogen chloride or hydrogen bromide at room temperature [cf., J. Org. Chem., 3, 26(1938)]. Also, hydrogen fluoride is highly reactive with halogenated olefins, depending upon their structures [cf., J. Phys. Chem., 44, 275(1940)]. Specifically, it reacts easily e.g., with 1,1-dichloroethylene at 65°C [cf., J. Am. Chem. Soc., 65, 1271(1943)]. Further, hydrogen halide [HX; X=F, Cl, Br or I] is generally known as an electrophilic reagent. It is considered that in the addition reaction of olefin with hydrogen halide, ionized $H^+$ initially attacks olefin and then $X^-$ reacts with carbonium cation formed as the intermediate. Hydrogen fluoride however reacts with a polyhalogenated-olefins, occurring predominant nucleophilic attack of $F^-$, thereby affording the desired product in good yield [J. Am. Chem. Soc., 82, 3091(1960)]. However, such nucleophilic reaction is not known for hydrogen chloride and hydrogen bromide. Accordingly, the reaction of 1,1-dihalogenoethylene with hydrogen chloride or hydrogen bromide whose reactivity is different from hydrogen fluoride is not suggested or predicted by the fact that 1,1-dihalogenoethylene as a kind of polyhalogenoolefins reacts easily with hydrogen fluoride.

Under these circumstances, the inventors of this invention have made various studies and have found unexpectedly the fact that when a 1,1-dichloroethylene is reacted with nitric acid or its salt and hydrogen chloride or hydrogen bromide or its salt, it provides at a high yield 1,1,1-trichloro-2-nitroethane (or 1-nitro-2,2,2-trichloroethane) in which a nitro group and a chlorine atom are introduced at the same time and at the specific positions.

3. Summary of the Invention

According to this invention, it provides

1) a process for the production of 1,1,1-trichloro-2-nitroethane which comprises reacting 1,1-dichloroethylene of the formula (I):

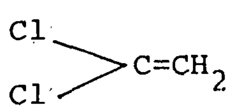

(I)

with nitric acid or its salt and hydrogen chloride or its salt to obtain a compound of the formula (II):

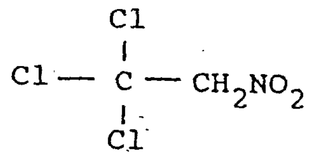

(II)

the reaction being carried out in an aqueous system to which an inert organic solvent may be added, at 0-100° C in a sealed vessel or at 0-40° C in an open system, and each of nitric acid and hydrogen chloride being used in 0.5 to 5 equivalents to the compound (I).

2) a process for the production of $\alpha$-unsaturatedamines which comprise subsequently reacting 1,1,1-trichloro-2-nitroethane of the formula (II) prepared according to the above 1) with an amino compound of the formula (III):

$$R^1\text{-NH-}C_nH_{2n}\text{-A} \quad \text{(III)}$$

wherein $R^1$ is a hydrogen atom, an $C_{1-4}$ alkyl, halo-$C_{1-4}$alkyl, mono- or di-$C_{1-4}$alkoxy-$C_{1-4}$ alkyl, $C_{7-9}$aralkyl, optionally substituted phenyl, mono- or di-$C_{1-4}$alkylamino or $C_{1-4}$alkoxy group, A is a 3- or 4-pyridyl, pyrazinyl, 2-, 4- or 5-thiazolyl or phenyl group which may be substituted by a halogen atom or a $C_{1-4}$alkyl, $C_{1-4}$alkylthio or $C_{1-4}$alkoxy group, and n is 0, 1 or 2, or its salt and an amino compound of the formula (IV):

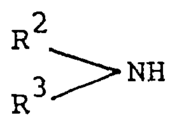

(IV)

,wherein $R_2$ is a hydrogen atom, or a $C_{1-4}$alkyl or $C_{7-9}$ aralkyl group, $R_3$ is a hydrogen atom, a $C_{1-5}$alkyl, halo-$C_{1-4}$alkyl, hydroxy-$C_{1-4}$alkyl, mono- or di-$C_{1-4}$alkoxy-$C_{1-4}$alkyl, mono- or di-$C_{1-4}$ alkylthio-$C_{1-4}$alkyl, di-$C_{1-4}$alkylamino-$C_{1-4}$alkyl, tri-$C_{1-4}$-alkylsilyl-$C_{1-4}$alkyl, $C_{2-4}$alkenyl, $C_{7-9}$aralkyl, optionally substituted phenyl, amino, di-$C_{1-4}$ alkylamino, pyridyl-$C_{1-2}$alkyl or thiazolyl-$C_{1-2}$alkyl group (pyridyl and thiazolyl ring may be substituted by a halogen atom), or $R^2$ and $R^3$ together with the adjacent nitrogen atom may form a 5 or 6-membered heterocylic ring which may contain an oxygen atom or another nitrogen atom, or its salt, to obtain a compound of the formula (V):

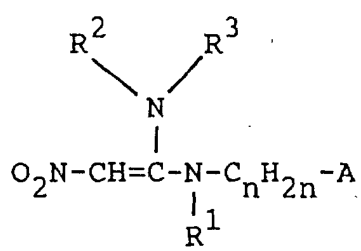

(V)

wherein $R^1$, $R^2$, $R^3$, A and n have the same meanings as defined above or its salt; and

3) a process for the production of $\alpha$-unsaturated-amines which comprises subsequently treating 1,1,1-trichloro-2-nitroethane of the formula (II) prepared according to the above 1) with a base to obtain a 1,1-dichloro-2-nitroethylene of the formula (VI) :

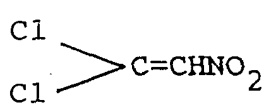

(VI) ,

and then reacting it with an amino compound of the formula (III):

$$R^1\text{-NH-}C_nH_{2n}\text{-A} \quad \text{(III)}$$

wherein $R^1$, A and n have the same meanings as defined in the above 2), or its salt and an amino compound of the formula (IV):

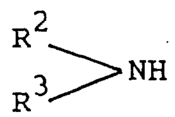

(IV)

wherein $R^2$ and $R^3$ have the same meanings as defined in the above 2), or its salt to obtain a compound of the formula (V):

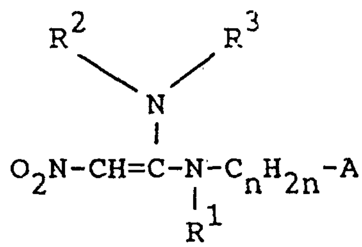

(V)

wherein $R^1$, $R^2$, $R^3$ A and n have the same meanings as mentioned above or its salt;

3. Preferred Embodiments of the Invention

With respect to $R_1$ of the above mentioned formulas,

the $C_{1-4}$alkyl may be methyl, ethyl, n-propyl, i-propyl, n-butyl, i-butyl, s-butyl or t-butyl, among which methyl, ethyl or n-propyl is preferable;
the halo-$C_{1-4}$alkyl may be monochloromethyl, dichloromethyl, trichloromethyl, monofluoromethyl, difluoromethyl, trifluoromethyl, 2-chloroethyl, 2,2,2-trichloroethyl, 2-fluoroethyl, 2,2,2-trifluoroethyl or 1,1,2,2-tetrafluoroethyl;
the mono- or di-$C_{1-4}$alkoxy-$C_{1-4}$alkyl group may be methoxymethyl, dimethoxymethyl, ethoxymethyl, 1- or 2-methoxyethyl, 2,2-dimethoxyethyl, 3-methoxypropyl or 3,3-dimethoxypropyl, among which methoxymethyl, dimethoxymethyl, 2-methoxyethyl or 2,2-dimethoxyethyl is preferable;
the $C_{7-9}$aralkyl group may be benzyl, 4-chlorobenzyl, 4-bromobenzyl, 4-methylbenzyl, phenethyl or 4-methylphenethyl;
the optionally substituted phenyl group may be phenyl or a phenyl substituted by one to four substituents of a halogen (e.g., fluorine, chlorine, bromine or iodine), $C_{1-3}$alkyl (e.g., methyl, ethyl or propyl), $C_{1-3}$alkoxy (e.g., methoxy, ethoxy or propoxy), amino, hydroxy, carboxy or sulfo;
the mono- di-$C_{1-4}$alkylamino group may be methylamino, ethylamino, n-propylamino, i-propylamino or n-butylamino, or dimethylamino, methylethylamino, diethylamino or di-n-propylamino;
the $C_{1-4}$alkoxy group may be methoxy, ethoxy, n-propoxy, i-propoxy, n-butoxy or i-butoxy.

Preferable examples of $R^1$ are hydrogen atom and a $C_{1-4}$alkyl group such as methyl, ethyl or propyl.
With respect to $R^2$, the $C_{1-4}$alkyl group and $C_{7-9}$aralkyl group mentioned in $R^1$ are applicable to these groups of $R^2$. Preferable examples of $R^2$ are hydrogen atom and a $C_{1-4}$alkyl group such as methyl, ethyl or propyl.
With respect to $R^3$, the $C_{1-5}$alkyl group includes the $C_{1-4}$alkyl groups examplified in $R^1$ and also amyl;

the halo $C_{1-4}$alkyl, mono- or di-$C_{1-4}$alkoxyalkyl, $C_{7-9}$aralkyl, optionally substituted phenyl and di-$C_{1-4}$alkylamino groups include the same ones examplified in $R^1$;
the hydroxy $C_{1-4}$alkyl group may be hydroxymethyl, 1- or 2-hydroxyethyl, or 3-hydroxypropyl;

the mono- or di-$C_{1-4}$alkylthio-$C_{1-4}$alkyl group may be methylthioethyl, ethylthioethyl, n-propylthioethyl methylthiopropyl, dimethylthiomethyl, diethylthiomethyl, dimethylthioethyl or dimethylthiopropyl;
the di-$C_{1-4}$alkylamino-$C_{1-4}$alkyl group may be dimethylaminoethyl, diethylaminomethyl or dimethyl aminopropyl;
the tri-$C_{1-4}$alkylsilyl-$C_{1-4}$alkyl group may be trimethylsilylmethyl, trimethylsilylethyl, trimethylsilylpropyl, triethylsilylmethyl or tri-n-propylsilylmethyl;
the $C_{2-4}$alkenyl group may be vinyl, allyl or isopropenyl;
the pyridyl-$C_{1-2}$alkyl group may be substituted by one to three halogens (e.g., Cl. Br or F) on the pyridine ring and includes e.g., (3-pyridyl)methyl, (6-chloro-3-pyridyl)methyl, (6-fluoro-3-pyridyl)methyl, (6-bromo-3-pyridyl)methyl and 1-(3-pyridyl)ethyl;
the thiazolyl-$C_{1-2}$alkyl group may be substituted by one to three halogens (e.g., Cl, Br or F) on the thiazole ring and includes e.g., (2-thiazolyl)methyl, (5-thiazolyl)methyl, (2-chloro-5-thiazolyl)methyl, (2-bromo-5-thiazolyl)methyl, (4-thiazolyl)methyl and 1-(5-thiazolyl)ethyl. Preferable examples of $R^3$ are hydrogen, or a $C_{1-4}$alkyl such as methyl, ethyl or propyl.

The 5- or 6-membered heterocyclic group which may contain an oxygen atom and another nitrogen atom (in addition to the adjacent nitrogen atom to $R_2$ and $R_3$) may be pyrrolidino, piperidino, morpholino, or 4-methylpiperazino.

With respect to A, the 3- or 4-pyridyl, pyrazinyl, 2-, 4- or 5-thiazolyl or phenyl group may be substituted by one to four substituents of a halogen, $C_{1-4}$alkyl, $C_{1-4}$alkylthio or $C_{1-4}$alkoxy. Here, the halogen may be fluorine, chlorine, bromine or iodine, the $C_{1-4}$alkyl or $C_{1-4}$alkoxy includes the same groups examplified in $R^1$ and the $C_{1-4}$ alkylthio may be methylthio, ethylthio, n-propylthio, i-propylthio, n-butylthio or i-butylthio. Examples of the 3- or 4-pyridyl groups which may be substituted are 3-pyridyl, 2- or 6-chloro-3-pyridyl, 5- or 6-bromo-3-pyridyl, 6-fluoro-3-pyridyl, 6-methoxy-3-pyridyl, 6-methyl-3-pyridyl, 5- or 6-trifluoromethyl-3-pyridyl, 2-methylthio-3-pyridyl, 2,6- or 5,6-dichloro-3-pyridyl, 4-pyridyl and 2,6-dichloro-4-pyridyl. Examples of the 2,4- or 5-thiazolyl groups which may be substituted are 2-thiazolyl, 4-thiazolyl, 5-thiazolyl, 2-chloro-5-thiazolyl, 2-bromo-5-thiazolyl, 2-fluoro-5-thiazolyl, 2-methyl-5-thiazolyl, 2-trifluoromethyl-5-thiazolyl, 2,4-dichloro-5-thiazolyl and 2-phenyl-5-thiazolyl. Examples of the pyrazinyl groups which may be substituted are 2-pyrazinyl, 5-chloro-2-pyradinyl, 5-bromo-2-pyrazinyl, 5-fluoro-2-pyradinyl, 5-methoxy-2-pyradinyl and 5-methyl-2-pyradinyl. Examples of the phenyl groups which may be substituted are phenyl, p-chlorophenyl, p-bromophenyl, p-fluorophenyl, p-tolyl and p-methoxyphenyl.

Preferable examples of A are 2-halogeno-5-thiazolyl such as 2-chloro-5-thiazolyl and 6-halogeno-3-pyridyl such as 6-chloro-3-pyridyl.

A preferable example of $C_n H_{2n}$ is $CH_2$. The starting material of this invention, i.e., 1,1-dichloroethylene, is commercially available at a low price.

According to this invention, 1,1-dichloroethylene (I) is reacted with nitric acid or its salt and hydrogen chloride or its salt to obtain a 1,1,1-trihalogeno-2-nitroethane (II). Each of nitric acid and hydrogen chloride is generally used in 0.5 to 5 equivalents, preferably 1.0 to 2.0 equivalents, more preferably 1.2 to 1.5 equivalents to the compound (I). In case of using a salt of hydrogen chloride, further addition of nitric acid enough to generate the hydrogen chloride is desirable to give a good result. Similarly, when a salt of nitric acid is used, further addition of hydrogen chloride which is necessary for generating nitric acid will achieve a good result. Examples of the salts of the hydrogen chloride or nitric acid are the alkaline metal salts, alkaline earth metal salts, and ammonium salt. Needless to say, it is possible to use the mixture of nitric acid and its salt or the hydrogen chloride and its salt. The hydrogen chloride can be used as it is but is conveniently used as its aqueous solution which is easy to handle, i.e., as hydrochloric acid. The concentration of each of nitric acid and the hydrogen chloride to be used in the reaction is suitably selected, as far as it does not impede the reaction. As nitric acid, 60% to 70% nitric acid which is available on the market or a fuming nitric acid can be used. About 35% hydrochloric acid which is available on the market can be used as hydrogen chloride. This hydrochloric acid and nitric acid can be used by diluting with water, although it may cause the delay of the reaction speed. The preferred initial concentration of each of the hydrogen chloride and nitric acid to be added in the reaction system is about 40% to 60%.

The reaction is usually carried out in an aqueous system, to which an inert organic solvent may be added. Examples of the organic solvents are those which are not easily mixable with water, such as hydrocarbons (e.g., hexane, petroleum ether, ligroin, cyclohexane, benzene, toluene, or xylene), ethers (e.g., diethyl ether or diisopropyl ether), esters (e.g., ethyl acetate) or halogenated hydrocarbons (e.g., chloroform, dichloromethane, carbon tetrachloride, 1,2-dichloroethane or 1,1-dichloroethylene as the starting material); those which are homogenerously mixable with water, such as nitriles (e.g., acetonitrile), tetrahydrofuran (hereinafter referred to as "THF") or dioxane.

The reaction can be conducted at 0 - 100°C in a sealed vessel but be conducted at 0 - 40°C, preferably 10 - 35°C in an open system. The reaction time is 5 minutes or more, preferably 0.5 to 48 hours. However, the reaction time of 5 minutes or more, preferably 0.5 to 5 hours is sufficient in case where the preferred concentrations of the hydrogen chloride and nitric acid and the preferred reaction temperature are selected.

The order of addition of the raw materials, i.e., the compound (I), nitric acid or its salt and the hydrogen chloride

or its salt in the reaction system can be optionally determined. That is, to the mixture of optional two kinds of the raw materials can be added the remaining raw material, or to one optional raw material can be added the remaining two raw materials at the same time. Alternatively, the three raw materials can be simultaneously mixed. We found the fact that nitric acid is reacted with the 1,1-dichloroethylene to produce the 1,1,1-trichloro-2-nitroethane. For instance, when 1,1-dichloroethylene is reacted with 70% nitric acid, it affords the mixture of 1,1,1-trichloro-2-nitroethane and 1,1,1,2-tetrachloroethane as main products, which however is not satisfied on the point of yield. On the other hand, hydrochloric acid is hardly reacted with the 1,1-dichloroethylene under the condition of the present invention. Thus, it will be understood that in accordance with the reaction of the present invention, the coexistence of nitric acid and the hydrogen chloride is required to prepare the 1,1,1-trichloro-2-nitroethane (II) in high yield from the 1,1-dichloroethylene.

The completion of the reaction can be detected by the stop of an exothermic reaction, or the conventional analysis with an instrument such as gas chromatography or NMR. The object compound (II) can be isolated by the conventional methods such as liquid separation, extraction and evaporation. The obtained 1,1,1-trichloro-2-nitroethane is industrially useful.

The 1,1,1-trichloro-2-nitroethane (II) is reacted with an amino compound of the formula (III):

$$R^1\text{-NH-}C_nH_{2n}\text{-A} \qquad \text{(III)}$$

wherein $R^1$, A and n have the same meanings as defined above, or its salt and an amino compound of the formula (IV):

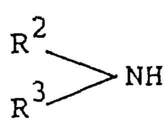

(IV)

wherein $R^2$ and $R^3$ have the same meanings as defined above, or its salt to provide an α-unsaturated amine of the formula (V):

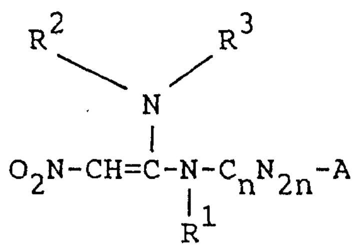

(V)

wherein $R^1$, $R^2$, $R^3$, A and n have the same meanings as defined above or its salt.

The reaction is preferably conducted in the presence of a base, in order to avoid a consumption of the amino compound by hydrogen chloride which is produced as by-product of this reaction. Although there is no limitation of the order of addition of the two amino compounds (III) and (IV), it is preferable to conduct at first the reaction of a secondary amine in case of the combination of primary and secondary amines. Accordingly, it is possible to react the compound (II) with the compound (III), subsequently with the compound (IV), and alternatively to react the compound (II) with the compound (IV) followed by reacting with the compound (III).

Suitable examples of the bases to be employed in the reaction are organic bases such as triethylamine, tri-n-propylamine, pyridine, collidine, quinoline, dimethylaniline, methyldicyclohexylamine, 1,5-diazabicyclo[4.3.0]non-5-ene, 1,4-diazabicyclo[2.2.2]-octane, 1,8-diazabicyclo[5.4.0]-7-undecene and 3,4-dihydro-2H-pyrido[1,2-a]-pyrimidin-2-one; inorganic bases such as sodium hydrogen carbonate, potassium hydrogen carbonate, sodium carbonate, potassium carbonate, sodium hydroxide, potassium hydroxide and calcium hydroxide; salts of carboxylic acids such as sodium acetate and potassium acetate. It is also possible to use as the base the amino compounds themselves which are used as the raw materials. The base is preferably used in 3 or more equivalents, to the compound (II), and there is no particular limitation in the timing of addition thereof as far as it does not impede the reacion.

The reaction is usually carried out in a solvent which does not impede the reaction. Suitable examples of the solvents are water; aliphatic hydrocarbons such as hexane, petroleum ether, ligroin and cyclohexane; aromatic hydrocarbons such as benzene, toluene, xylene and chlorobenzene; alcohols such as methanol, ethanol, n-propanol, isopropanol, n-butanol and tert-butanol; halogenated hydrocarbons such as chloroform, dichloromethane, carbon tetrachloride and 1,2-dichloroethane, ethers such as diethyl ether, diisopropyl ether, dioxane and tetrahydrofuran; ketones such as acetone and methyl ethyl ketone; nitriles such as acetonitrile and propionitrile; amides such as dimethylformamide and dimethylacetamide; esters such as methyl acetate, ethyl acetate and butyl acetate, and sulfoxides such as dimethyl sulfoxide.

The reaction temperature can be selected from the range of -80°C or higher, but is generally -40°C or higher, preferably -40°C to 120°C, more preferably -20°C to 50°C. The reaction will be completed within a relatively short time of 5 minutes to 5 hours. The object compound (V) after completion of the reaction can be isolated by the conventional methods such as filtration, concentration, extraction and column chromatography.

Also, the α-unsaturated amine (V) or its salt can be obtained by treating a 1,1,1-trichloro-2-nitroethane (II) with a base to obtain a 1,1-dichloro-2-nitroethylene (VI) and then reacting the compound (VI) with or without isolation with the amino compounds (III) and (IV) or salts thereof.

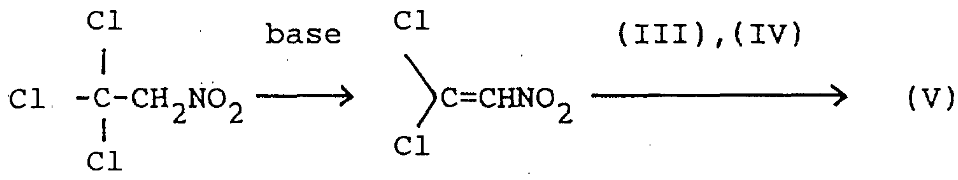


(II) (VI)

The first reaction for obtaining the compound (VI) from the compound (II) and the base can be conducted with or without solvent. Suitable examples of the solvents to be employed are water; aliphatic hydrocarbons such as hexane, petroleum ether, ligroin and cyclohexane; aromatic hydrocarbons such as benzene, toluene, xylene and chlorobenzene; alcohols such as methanol, ethanol, n-propanol, isopropanol, n-butanol and tert-butanol; halogenated hydrocarbons such as chloroform, dichloromethane, carbon tetrachloride and 1,2-dichloroethane; ethers such as diethyl ether, diisopropyl ether, dioxane and tetrahydrofuran; ketones such as acetone and methylethylketone; nitriles such as acetonitrile and propionitrile; amides such as dimethylformamide and dimethylacetamide; esters such as methyl acetate, ethyl acetate and butyl acetate; and sulfoxides such as dimethyl sulfoxide.

Examples of the bases to be employed in the reaction are organic bases such as triethylamine, tri-n-propylamine, pyridine, collidine, quinoline, dimethylaniline, methyldicyclohexylamine, 1,5-diazabicyclo[4.3.0]-non-5-ene, 1,4-diazabicyclo-[2.2.2]octane, 1,8-diazabicyclo[5.4.0]-7-undecene and 3,4-dihydro-2H-pyrido[1.2-a]pyrimidin-2-one; inorganic bases such as sodium hydrogen carbonate, potassium hydrogen carbonate, sodium carbonate, potassium carbonate, sodium hydroxide, potassium hydroxide and calcium hydroxide; salts of carboxylic acid salts such as sodium acetate and potassium acetate. Such bases are used in 1 to 5 equivalents, preferably 1 to 2 equivalents, to the compound (II). The reaction temperature can be selected in the range of -80°C or higher, but is generally -40°C or higher, preferably -40°C to 120°C, more preferably -20°C to 50°C.

The completion of the reaction can be detected e.g., by gas chromatography or NMR. The reaction will be completed within a relatively short time such as 15 minutes to 5 hours. For the isolation of the resulting product (VI), the conventional method such as extraction, filtration, concentration or evaporation is suitably utilized.

In the second reaction for obtaining the compound (V) from the compound (VI), the order of the reaction of two kinds of the amino compounds (III) and (IV) can be freely selected and, in case of the combination of a primary amine and a secondary amine, it is preferable to allow to firstly react the secondary amine. Any one of the compounds (III) and (IV) can be firstly used to react.

The reaction is preferably conducted in a solvent, to which those exemplified in the above mentioned first reaction are applicable. The reaction is preferably conducted in the presence of such base, to which those mentioned in the above mentioned first reaction are applicable. The base is used in 2 to 5 equivalents, preferably 2 to 3 equivalents, to the compound (VI). As occasion demands, it is also possible to use as such base the amino compound itself which is employed as the raw material. The reaction temperature can be chosen from the range of -80°C or higher, preferably -40°C to 120°C but is generally -40°C or higher, especially -20°C to 50°C. The reaction time is relatively short and is 5 minutes to 5 hours.

The isolation of the resulting compound (V) after completion of the reaction can be carried out by the conventional method such as filtration,concentration, extraction and column chromatography.

In the above reactions, it is especially preferable to use secondary amines or salts thereof as either one or both of two kinds of the amino compounds (III) and (IV).

Examples of salts of the amino compounds (III) and (IV) are the salts with an inorganic acid such as hydrochloric acid, sulfuric acid or nitric acid; an organic acid such as benzenesulfonic acid; and a base such as sodium, potassium or lithium (e.g., $C_{1-4}$alkyl-N-(Na)$C_nH_{2n}$-A).

The amino compounds (III) and (IV) can be easily produced by the known methods, as described e.g., in Organic Functional Group Preparations, Academic Press Vol 1, Chapter 13 (1968) and Vol 3, Chapter 10 (1972); Survey of Organic Syntheses, Wiley-Inter-Science (1970), Chapter 8, or analogous methods thereto.

Specific examples of the α-unsaturated-amines (V) as produced are shown in the Table 1.

Table 1

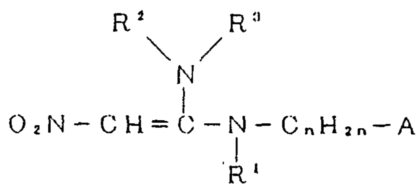

| Compound NO. | $R^1$ | $R^2$ | $R^3$ | $C_nH_{2n}$ | A | Melting point |
|---|---|---|---|---|---|---|
| 1 | H | H | Me | $CH_2$ | 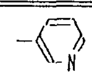 | 159-160 |
| 2 | H | H | Et | $CH_2$ | 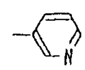 | 161-162 |
| 3 | H | H | i-Pr | $CH_2$ | 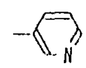 | 148-150 |
| 4 | H | H | n-Bu | $CH_2$ | 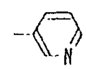 | 110-112 |
| 5 | H | H | Allyl | $CH_2$ | 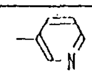 | 114-115 |
| 6 | H | H | n-$C_5H_{13}$ | $CH_2$ | 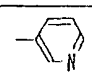 | 97-98 |
| 7 | H | H | 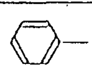 | $CH_2$ | 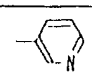 | 217-218 |
| 8 | H | H | H | $CH_2$ | 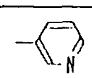 | 177-178 |
| 9 | H | H | n-PrS$(CH_2)_2$ | $CH_2$ | 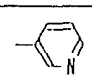 | 93-94 |
| 10 | H | H | $Me_2N(CH_2)_2$ | $CH_2$ | 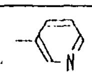 | 110-111 |
| 11 | H | H | $HO(CH_2)_2$ | $CH_2$ | 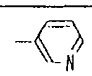 | 161-163 |
| 12 | H | H | $MeO(CH_2)_2$ | $CH_2$ | 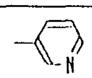 | 108-109 |
| 13 | H | H | $(MeO)_2CHCH_2$ | $CH_2$ | 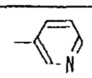 | 96-98 |

Table 1 (continued)

| Compound No. | $R^1$ | $R^2$ | $R^3$ | $C_nH_{2n}$ | A | Melting point |
|---|---|---|---|---|---|---|
| 14 | H | H | $CF_3CH_2$ | $CH_2$ | 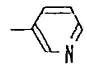 | 164-165 |
| 15 | H | H | $Me_3SiCH_2$ | $CH_2$ | 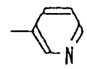 | 156-157 |
| 16 | H | H | $NH_2$ | $CH_2$ | 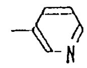 | 176-177 decomposition |
| 17 | H | Me | Me | $CH_2$ | 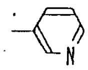 | 68-70 |
| 18 | H | $-(CH_2)_4-$ | | $CH_2$ | 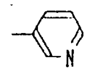 | 103-105 |
| 19 | H | $-(CH_2)_2-N(Me)-(CH_2)_2-$ | | $CH_2$ | 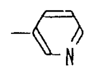 | oily form |
| 20 | H | $-(CH_2)_2-O-(CH_2)_2-$ | | $CH_2$ | 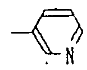 | 102-103 |
| 21 | H | $-(CH_2)_5-$ | | $CH_2$ | 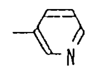 | 106-108 |
| 22 | H | H | $Me_2N$ | $CH_2$ | 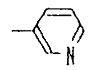 | 158-159 |
| 23 | Me | H | H | $CH_2$ | 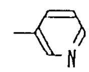 | 158-159 |
| 24 | Me | H | Me | $CH_2$ | 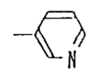 | 86-87 |
| 25 | H | H | Me | $CH_2$ | 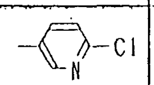 | 181-183 |
| 26 | Me | H | Me | $CH_2$ | 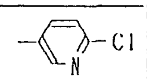 | 103-104 |
| 27 | Et | H | Me | $CH_2$ | 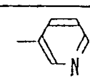 | oily form |
| 28 | $(MeO)_2CHCH_2$ | H | Me | $CH_2$ | 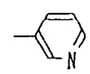 | oily form |
| 29 | Me | H | Et | $CH_2$ | 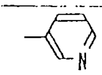 | oily from |

Table 1 (continued)

| Compound No. | $R^1$ | $R^2$ | $R^3$ | $C_nH_{2n}$ | A | Melting point |
|---|---|---|---|---|---|---|
| 30 | Me | H | n-Bu | $CH_2$ | 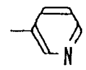 | oily form |
| 31 | $MeO(CH_2)_2$ | H | Me | $CH_2$ | 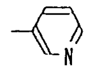 | oily form |
| 32 | Me | H | Allyl | $CH_2$ | 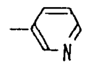 | oily form |
| 33 | Me | H | i-Pr | $CH_2$ | 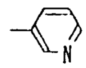 | 119-121 |
| 34 | Me | H | 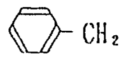 | $CH_2$ | 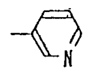 | oily form |
| 35 | Me | H | Me | $CH_2$ | 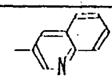 | 145-147 |
| 36 | Me | H | Me | Me<br>CH | 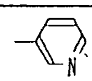 | oily form |
| 37 | $Me_2N$ | H | Me | $CH_2$ | 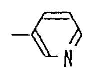 | 109-110 |
| 38 | n-Pr | H | Me | $CH_2$ | 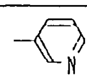 | oily form |
| 39 | n-Bu | H | Me | $CH_2$ | 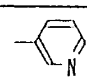 | oily form |
| 40 | 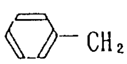 | H | Me | $CH_2$ | 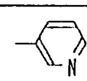 | 118-119 |
| 41 | Me | H | H | $CH_2$ | 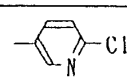 | 206-207 |
| 42 | H | Me | Me | $CH_2$ | 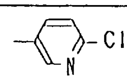 | 124-125 |
| 43 | H | H | Me | $CH_2$ | 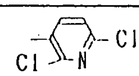 | 211-213 decomposition |
| 44 | Me | H | H | $CH_2$ | 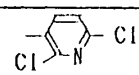 | 214-215 decomposition |
| 45 | i-Pr | H | H | $CH_2$ | 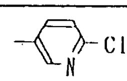 | powder |

Table 1 (continued)

| Compound No. | $R^1$ | $R^2$ | $R^3$ | $C_nH_{2n}$ | A | Melting point |
|---|---|---|---|---|---|---|
| 46 | H | Me | Et | $CH_2$ | 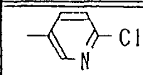 | 87-88 |
| 47 | H | H | $NH_2$ | $CH_2$ | 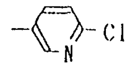 | 188-190 decomposition |
| 48 | H | H | $Me_2N$ | $CH_2$ | 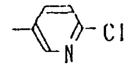 | 170-172 |
| 49 | Me | H | Me | $CH_2CH_2$ | 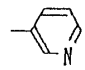 | oily form |
| 50 | Me | Me | Me | $CH_2$ | 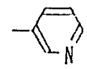 | 103-105 |
| 51 | Me | Me | 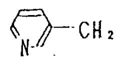 | $CH_2$ | 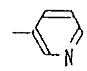 | oily form |
| 52 | Me | Me | Me | $CH_2$ | 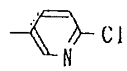 | 110-112 |
| 53 | Et | H | H | $CH_2$ | 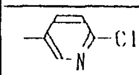 | 159-161 |
| 54 | Et | H | Me | $CH_2$ | 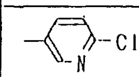 | 83-84 |
| 55 | Me | H | Me | $CH_2$ | 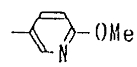 | 77-78 |
| 56 | Me | H | Me | $CH_2$ | 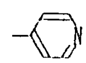 | 145-146 |
| 57 | Me | H | Me | $CH_2$ | 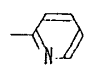 | 96-97 |
| 58 | MeO | H | Me | $CH_2$ | 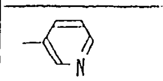 | 100-101 |

Table 1 (continued)

| Compound No. | $R^1$ | $R^2$ | $R^3$ | $C_nH_{2n}$ | A | Melting point |
|---|---|---|---|---|---|---|
| 59 | Me | H | $MeO(CH_2)_2$ | $CH_2$ | 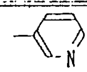 | 55-57 |
| 60 | Me | H | Me | $CH_2$ | 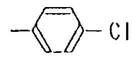 | 98-99 |
| 61 | H | H | H | $CH_2$ | 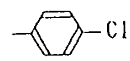 | 215-216 decomposition |
| 62 | H | H | Me | $CH_2$ | 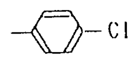 | 219-220 decomposition |
| 63 | H | Me | Me | $CH_2$ | 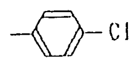 | 133-135 |
| 64 | Me | H | Me | $CH_2$ | 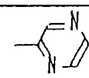 | 132-133 |
| 65 | Me | H | $Me_2N$ | $CH_2$ | 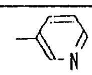 | 80-82 |
| 66 | n-Pr | H | H | $CH_2$ | 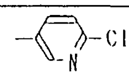 | 185-186 decomposition |
| 67 | n-Pr | H | Me | $CH_2$ | 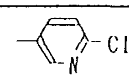 | 102-103 |
| 68 | i-Pr | H | Me | $CH_2$ | 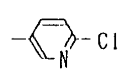 | 119-120 |
| 69 | Me | H | Me | --- | 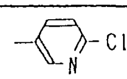 | 108-109 |
| 70 | Me | H | Me | --- | 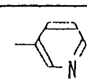 | 113-114 |
| 71 | Me | H | Et | $CH_2$ | 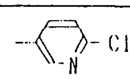 | 132-133 |
| 72 | Me | H | Me | $CH_2$ | 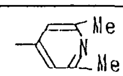 | 131-133 |
| 73 | Me | H | Me | $CH_2$ | 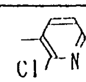 | 106-113 |

Table 1 (continued)

| Compound No. | $R^1$ | $R^2$ | $R^3$ | $C_nH_{2n}$ | A | Melting point |
|---|---|---|---|---|---|---|
| 74 | Me | H | Me | --- | 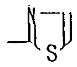 | 155-156 |
| 75 | Me | H | Me | --- | 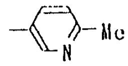 | 120-121 |
| 76 | Me | H | Et | --- | 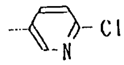 | 118-119 |
| 77 | Me | H | Me | $CH_2$ | 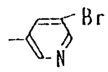 | 116-117 |
| 78 | Me | H | Me | $CH_2$ | 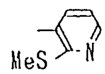 | 131-132 |
| 79 | Me | H | Me | $CH_2$ | 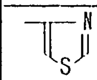 | 155-156 |
| 80 | H | H | 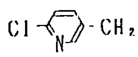 | $CH_2$ | 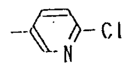 | 238-240 decomposition |
| 81 | Et | H | Me | --- | 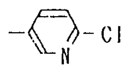 | 95-95 |
| 82 | n-Pr | H | Me | --- | 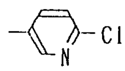 | 94-95 |
| 83 | n-Bu | H | Me | --- | 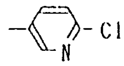 | 87-88 |
| 84 | Et | H | Et | --- | 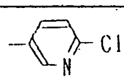 | 105 |
| 85 | Me | H | Me | --- | 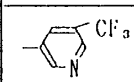 | 114-115 |
| 86 | Me | H | n-Pr | --- | 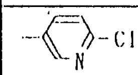 | oily form |
| 87 | H | H | Me | --- | 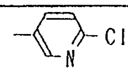 | 185 decomposition |
| 88 | Me | H | Me | $CH_2$ | 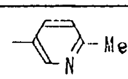 | 102-103 |
| 89 | Me | H | Me | $CH_2$ | 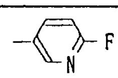 | 100-100.5 |

Table 1 (continued)

| Compound No. | $R^1$ | $R^2$ | $R^3$ | $C_nH_{2n}$ | A | Melting point |
|---|---|---|---|---|---|---|
| 90 | Et | H | Me | $CH_2$ | 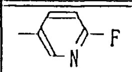 | oily form |
| 91 | Me | H | Me | $CH_2$ | 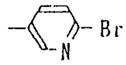 | 130-131 |
| 92 | Et | H | Me | $CH_2$ | 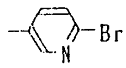 | 79-80 |
| 93 | Me | H | Me | $CH_2$ | 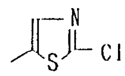 | 131-133 |
| 94 | Et | H | Me | $CH_2$ | 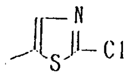 | 110-112 |
| 95 | H | Me | Me | $CH_2$ | 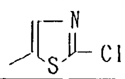 | 101-102 |
| 96 | H | H | 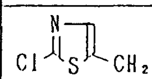 | $CH_2$ | 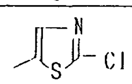 | 211 decomposition |
| 97 | H | H | Me | $CH_2$ | 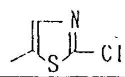 | 181 decomposition |
| 98 | H | Me | Me | --- | 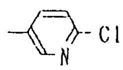 | 122-123 |
| 99 | Me | H | Me | --- | 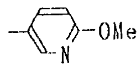 | 131-132 |
| 100 | H | H | Me | $CH_2$ | 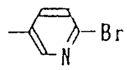 | 184-186 decomposition |
| 101 | H | Me | Me | $CH_2$ | 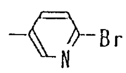 | 158-159 |
| 102 | Me | H | H | $CH_2$ | 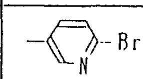 | 206-207 |
| 103 | $CF_3CH_2$ | H | Me | $CH_2$ | 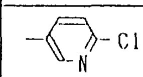 | 110-111 |
| 104 | H | H | Me | $CH_2$ | 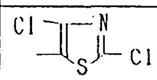 | 182-184 decomposition |
| 105 | H | H | Me | $CH_2$ | 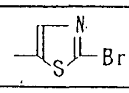 | 167-169 decomposition |

Table 1 (continued)

| Compound No. | $R^1$ | $R^2$ | $R^3$ | $C_nH_{2n}$ | A | Melting point |
|---|---|---|---|---|---|---|
| 106 | H | Me | Me | $CH_2$ | 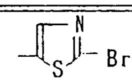 | 125 decomposition |
| 107 | Et | Me | Me | $CH_2$ | 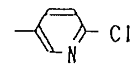 | oily form |
| 108 | $CH_2FCH_2$ | H | Me | $CH_2$ | 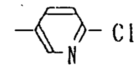 | 78-79 |
| 109 | $CH_2FCH_2$ | Me | Me | $CH_2$ | 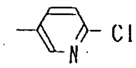 | 90-91 |
| 110 | $CH_2FCH_2$ | H | Me | $CH_2$ | 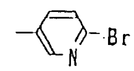 | |
| 111 | $CH_2FCH_2$ | Me | Me | $CH_2$ | 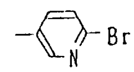 | |
| 112 | $CH_2FCH_2$ | H | Me | $CH_2$ | 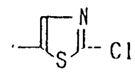 | |
| 113 | Me | Me | Me | $CH_2$ | 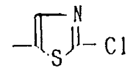 | |
| 114 | Et | Me | Me | $CH_2$ | 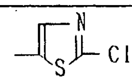 | |
| 115 | Me | H | Me | $CH_2$ | 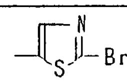 | |
| 116 | Et | H | Me | $CH_2$ | 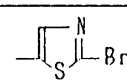 | |
| 117 | $CH_2FCH_2$ | Me | Me | $CH_2$ | 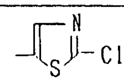 | |
| 118 | Me | Me | Me | $CH_2$ | 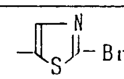 | |
| 119 | Et | Me | Me | $CH_2$ | 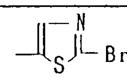 | |
| 120 | $ClCH_2$ | H | Me | $CH_2$ | 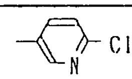 | |
| 121 | $ClCH_2$ | H | Me | $CH_2$ | 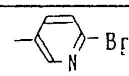 | |

Table 1 (continued)

| Compound No. | $R^1$ | $R^2$ | $R^3$ | $C_nH_{2n}$ | A | Melting point |
|---|---|---|---|---|---|---|
| 122 | $ClCH_2$ | H | Me | $CH_2$ | 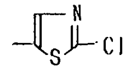 | |
| 123 | $CH_2FCH_2$ | H | Me | $CH_2$ | 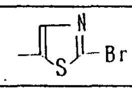 | |
| 124 | $CH_2FCH_2$ | Me | Me | $CH_2$ | 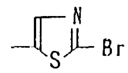 | |
| 125 | $CF_3CH_2$ | H | Me | $CH_2$ | 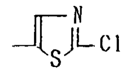 | |
| 126 | $CF_3CH_2$ | Me | Me | $CH_2$ | 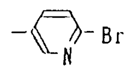 | |
| 127 | Me | Me | Me | $CH_2$ | 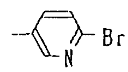 | |
| 128 | Et | Me | Me | $CH_2$ | 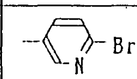 | |
| 129 | H | H | Me | $CH_2$ | 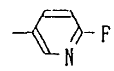 | |
| 130 | H | Me | Me | $CH_2$ | 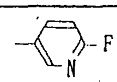 | |
| 131 | Me | Me | Me | $CH_2$ | 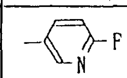 | |
| 132 | Et | Me | Me | $CH_2$ | 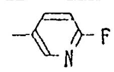 | |

Table 1 (continued)

| Compound No. | $R^1$ | $R^2$ | $R^3$ | $C_nH_{2n}$ | A | Melting point |
|---|---|---|---|---|---|---|
| 133 | CHO | H | Me | $CH_2$ | 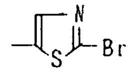 | |

*1 exists as 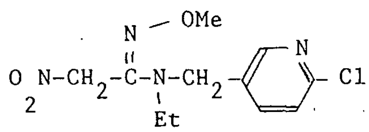

*2 exists as 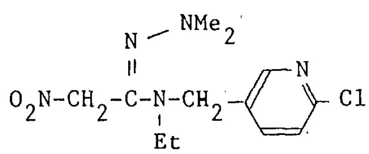

In the above table, Me, Et, n-Pr, i-Pr and n-Bu represent $CH_3$-, $CH_3CH_2$-, $CH_3CH_2CH_2$-,

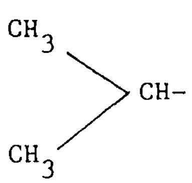 and $CH_3CH_2CH_2CH_2$-,

respectively.

When the compound (V) is obtained in the free form, it may be converted into its salt form by the conventional method and, on the contrary, the salt when obtained may be converted into the corresponding free form by the conventional method. That is, when the compound (V) has a basic group or moiety in the parts of $R^1$, $R^3$ and A, it may form the acid addition salt, e.g., the hydrochloride, hydrobromide, hydroiodide, nitrate, sulfate, phosphate, acetate, benzoate, maleate, fumalate, succinate, tartarate, citrate, oxalate, glyoxylate, aspartate, methanesulfonate, methanedisulfonate, 1,2-ethanedisulfonate or benzenesulfonate. Also, the compound (V) may form an inner salt which is also

included in this invention.

The compounds (V) can exist as steroisomers and tautomers, and such isomers and mixtures thereof are also included in this invention.

The compounds (V) or their salts are effective in preventing sanitary or horticultural insect pests and animal and plant parasites and can exert potent insecticidal activities when they are directly contacted with insects, e.g., by applying to their living animals or plants. An interesting characteristic property of the compounds (V) or their salts is found in that potent insecticidal activities can be achieved by once absorbing the compounds in plants through their root, leave or stem which are then sucked or bitten by insects or contacted with insects. Such property is advantageous for preventing suctorial type or mandible type insecticides. Moreover, the compounds (V) and their salts possess safe and advantageous properties as agents for preventing agricultural injurious insects, such as no substantial damage on plants and less toxicity against fishes.

Specifically, the preparations containing the compounds (V) or their salts are especially effective in preventing Hemiptera injurious insects such as Eurydema rugosum, Scotinophara lurida, Riptortus clavatus, Stephanitis nashi, Laodelphax striatellus, Nilaparvate lugens, Nephotettix cincticeps, Unaspis yanonensis, Aphis glycines, Lipaphis erysimi, Brevicoryne brassicae, Aphis gossypii, Sogattela furcifera, Nezara viridula, Trialeurodes vaporariorum, Myzus persicae, Pseudococcus comstocki, Aphis promi Nezara spp, Cimex lectularius and Psylla spp; Lepidoptera injurious insects such as Spodoptera litura, Plutella xylostella, Pieris rapae crucivora, Chilo suppressalis, Autographa nigrisigna, Helicoverpa assulta, Pseudaletia separata, Mamestra brassicae, Adoxophyes orana fasciata, Notarcha derogata, Cnaphalocrocis medinalis and Phthorimaea operculella; Coleoptera injurious insects such as Epilachna vigintioctopunctata, Aulacophora femoralis, Phyllotreta striotata, Oulema oryzae and Echinocnemus squameus; Diptera injurious insects such as Musca domestica, Culex pipiens pallens, Tabanus trigonus, Delia antiqua and Delia platura; Orthosptera injurious insects such as Locusta migratoria and Gryllotalpa africana; Dictyoptera injurious insects such as Blattella germanica and Periplaneta fuliginosa; Tetranychidae such as Tetranychus urticae, Panonychus citri, Tetranychus kanzawai, Tetranychus cinnabarinus, Panonychus ulmi and Aculops pelekassi; and Nematodes such as Aphelenchoides besseyi.

The compounds (V) or their salts can be used as insecticides or miticides in any application form suited for general agricultural chemicals. That is, one, two, or more than two kinds of the compounds (V) or their salts are used in the form of preparation such as emulsifiable concentrates, oil solutions, wettable powders, dusts, granules, tablets, sprays or ointments, according to the purpose of use, by dissolving or dispersing them in suitable liquid carriers, or mixing them with or absorbing them on suitable solid carriers. These preparations may contain, if necessary, emulsifying agent, suspending agent, spreading agent, penetrating agent, wetting agent, thickening agent or stabilizer, and can be prepared by any conventional method known per se.

The rate of the compound (V) or a salt thereof contained in an insecticidal preparation is suitably 10 to 90% by weight in the case of emulsifiable concentrates or wettable powders, 0.1 to 10% by weight in the case of oil solution or dust and 1 to 20% by weight in the case of granules. However, such concentration may be changed properly, depending on the purpose of use. Emulsifiable concentrates or wettable powders are suitably diluted or extended (for example, to 10 to 100000 times) with water, on the occasion or use, and then scattered.

Suitable examples of the liquid carriers (solvents) include solvents such as water, alcohols (for example, methanol, ethanol, n-propanol, isopropanol or ethylene glycol), ketones (for example, acetone or methyl ethyl ketone), ethers (for example, dioxane, tetrahydrofuran, ethylene glycol monomethyl ether, di-ethylene glycol monomethyl ether or propylene glycol monomethyl ether), aliphatic hydrocarbons (for example, kerosine, kerosene oil, fuel oil or machine oil), aromatic hydrocarbons (for example, benzene, toluene, xylene, solvent naphtha or methylnaphthalene), halogenated hydrocarbons (for example, dichloromethane, chloroform or carbon tetrachloride), acid amides (for example, dimethylformamid or dimethyl acetamide), esters (for example, ethyl acetate, butyl acetate or fatty acid glycerol ester ) or nitriles (for example, acetonitrile or propionitrile). These solvents are used individually or as a suitable mixture of two, or more, of them.

Suitable examples of the solid carriers (diluents or dust carrier) include vegetable powder (for example, soybean meal, tobacco meal, wheat flour or wood flour), mineral powders (for example, clays such as kaolin, bentonite, or acid clay, talcs such as talc powder or pyrophyllite powder), silicas (for example, diatomaceous earth or mica powder), aluminas, sulfur powder or active carbon. They are used individually or as a suitable mixture of two, or more, of them.

Also, suitable examples of bases for ointments include polyethylene glycol, pectin, polyalcohol esters of higher aliphatic acids (for example, glycerin mono-stearate), cellulose derivatives (for example, methyl cellulose), sodium alginate, bentonite, higher alcohols, polyalcohos (for example, glycerin), vaseline, white petrolatum, liquid paraffin, lard, various vegetable oils, lanolin, dehydrated lanolin, hard oil or resins. They are used individually, or as a suitable mixture of two, or more, of them or together with surface active agents mentioned below.

As surface active agents used as the emulsifying agent, spreading agent, penetrating agent or dispersing agent, nonionic or anionic surface active agents such as soaps; polyoxyethylene alkyl aryl ethers (e.g., Noigen® and E.A 142® from Dai-ichi Kogyo Seiyaku K.K., Japan, and Nonal® from Toho Chemical, Japan); alkyl sulfates (e.g., Emal

10® and Emal 40® from Kao K.K., Japan); alkyl sulfonates (e.g., Neogen® and Neogen T® from Dai-ichi Kogyo Seiyaku K.K. and Neopellex® from Kao K.K.); polyethylene glycol ethers (e.g., Nonipol 85® , Nonipol 100® , Nonipol 160® from Sanyo Kasei K.K., Japan); or polyhydric alcohol esters (e.g., Tween 20® and Tween 80® from Kao K.K.) are used, if necessary.

The compounds (V) or their salts can also be used, as occasion demands, in combination with or as an admixture with other insecticides (for example, pyrethroid insecticides, organophosphorus insecticides, carbamate insecticides or natural insecticides), acaricides, nematicides, herbicides, plant hormones, plant growth regulators, fungicides (for example, copper fungicides, organic chlorine fungicides, organic sulfur fungicides or phenol fungicides), synergistic agents, attractants, repellents, pigments and/or fertilizers.

The insecticidal or miticidal composition comprising the compound (V) or its salt of the present invention is an excellent agricultural product having fairly low toxicity and good safety. It can be used in a similar way to the conventional insecticidal or miticidal composition and can exert excellent effects in comparison with the conventional composition. For example, the insecticidal or miticidal composition of the present invention can be applied to the target insects, by treatment in nursery box, application for stem and leaf of crop, spraying for insects, application in water of a paddy field or soil treatment of a paddy field. The amount of application may broadly vary depending on the season, place and method of application, and so forth. However, the active ingredient (the compound (V) or its salt) is used in general, in an amount of 0.3g to 3,000g, preferably 50g to 1,000g per hectare. When the insecticidal composition of the present invention is in a wettable powder, it can be used by diluting it so as to be 0.1 - 1000 ppm, preferably 10 - 500 ppm as the final concentration of the active ingredient.

Activity

As will be clear from the following tests, the compounds (I) and salts thereof possess excellent insecticidal activities.

Test Example 1 (Effect against Nilaparvata lugens)

5mg of each of test compounds (shown by Compound No. obtained in Example as stated hereinafter) was dissolved in 0.5ml of acetone containing Tween 20® and diluted to a predetermined concentration (500 ppm) by addition of Dyne® (a spreader produced by Takeda Chemical Industries, Ltd. of Japan) diluted 3000 times with water. The solution at a rate of 10 ml/pot was sprayed on leaf and stem of rice seedlings at the second leaf stage raised in a nursery box. The treated rice seedlings were put into a test tube containing water at the bottom, to which 10 larvae at 3 instar of Nilaparvata lugens were released. After being sealed with an aluminum stopper, the test tube was kept in an incubator adjusted to 25°C. Death number was counted 7 days after release. The mortality rate was calculated by the following formula and shown in Table 2.

$$\text{Mortality (\%)} = \frac{\text{the number of dead insects}}{\text{the number of insects released}} \times 100$$

Table 2

| Compound No. | Mortality (%) |
|---|---|
| 1 | 100 |
| 2 | 100 |
| 5 | 100 |
| 10 | 100 |
| 12 | 100 |
| 15 | 100 |
| 16 | 100 |
| 17 | 100 |
| 18 | 100 |
| 22 | 100 |
| 23 | 100 |
| 24 | 100 |
| 25 | 100 |

Table 2 (continued)

| Compound No. | Mortality (%) |
|---|---|
| 26 | 100 |
| 27 | 100 |
| 28 | 100 |
| 29 | 100 |
| 30 | 100 |
| 31 | 100 |
| 33 | 100 |
| 34 | 100 |
| 36 | 100 |
| 37 | 100 |
| 38 | 100 |
| 39 | 100 |
| 40 | 100 |
| 41 | 100 |
| 42 | 100 |
| 43 | 100 |
| 45 | 100 |
| 46 | 100 |
| 47 | 100 |
| 48 | 100 |
| 49 | 100 |
| 50 | 100 |
| 51 | 100 |
| 52 | 100 |
| 53 | 100 |
| 54 | 100 |
| 55 | 100 |
| 56 | 100 |
| 58 | 100 |
| 59 | 100 |
| 60 | 100 |
| 62 | 100 |
| 63 | 100 |
| 64 | 100 |
| 66 | 100 |
| 67 | 100 |
| 68 | 100 |
| 69 | 100 |
| 70 | 100 |
| 71 | 100 |
| 75 | 100 |
| 76 | 100 |
| 77 | 100 |
| 79 | 100 |
| 80 | 100 |
| 81 | 100 |
| 82 | 100 |
| 83 | 100 |
| 84 | 100 |
| 86 | 100 |

Table 2 (continued)

| Compound No. | Mortality (%) |
|---|---|
| 87 | 100 |
| 88 | 100 |
| 89 | 100 |
| 90 | 100 |
| 91 | 100 |
| 92 | 100 |
| 93 | 100 |
| 94 | 100 |
| 95 | 100 |
| 96 | 100 |
| 97 | 100 |
| 98 | 100 |
| 99 | 100 |
| 100 | 100 |
| 101 | 100 |
| 102 | 100 |
| 103 | 100 |
| 105 | 100 |
| 106 | 100 |
| 107 | 100 |
| 108 | 100 |
| 109 | 100 |
| 111 | 100 |
| 112 | 100 |
| 113 | 100 |
| 133 | 100 |
| 115 | 100 |
| 116 | 100 |

Table 1 clearly reveals that the compounds (V) or salts thereof have an excellent insecticidal effect on Nilaparvate lugens.

Reference Example

1,1-Dichloroethylene 4.85g (0.05 mol) was dropwise added to 5.40g (0.06 mol) of 70% nitric acid at 23 - 27°C with stirring. After stirring for an hour at 23 - 25°C, the lower layer of the reaction mixture was collected, added with 10ml of ethyl ether and washed with 10ml of water. Then, the mixture was dried over magnesium sulfate and concentrated to obtain 6.21g of a crude product, which was found to contain 33% of 1,1,1-trichloro-2-nitroethane and 8% of 1,1,1,2-tetrachloroethane by NMR measurement.

Example 1.

To the mixture of 5.6g of 36% hydrochloric acid (0.055 mol) and 4.5g (0.05 mol) of 70% nitric acid (d 1.42) were dropwise added 4.85g (0.05 mol) of 1,1-dichloroethylene in 15 minutes at 23 - 26 °C. The reaction mixture was stirred at the same temperature for an hour and then extracted with chloroform. The chloroform layer was washed with water, dried over magnesium sulfate and concentrated to yield 6.4g (71.7%) of 1,1,1-trichloro-2-nitroethane. bp. 82.5-84°C/32 mbar (24mmHg).

Example 2.

7.6g (0.075 mol) of 36% hydrochloric acid, 4.5g (0.05 mol) of 70% nitric acid and 4.85g (0.05 mol) of 1,1-dichloroethylene were treated in the same way as in Example 1 to obtain 1,1,1-trichloro-2-nitroethane. Yield : 6.2g (69.5%).

Example 3.

7.6g (0.075 mol), of 36% hydrochloric acid, 6.8g (0.075 mol) of 70% nitric acid and 4.85g (0.05 mol) of 1,1-dichloroethylene were treated in the same way as in Example 1 to obtain 1,1,1-trichloro-2-nitroethane. Yield : 5.4g (60.5%).

Example 4.

To the mixture of 4.5g (0.05 mol) of 70% nitric acid and 4.85g (0.05 mol) of 1,1-dichloroethylene were dropwise added, 5.6g (0.055 mol) of 36% hydrochloric acid in 15 minutes at 23 - 26°C. The reaction mixture was stirred at the same temperature and then extracted with chloroform, the chloroform layer was dried over magnesium sulfate and concentrated to obtain 1,1,1-trichloro-2-nitroethane. Yield : 5.7g (63.9%).

Example 5

To the mixture of 5.6g (0.055 mol) of 36% hydrochloric acid and 4.85g (0.05 mol) of 1,1-dichloroethylene were dropwise added, 4.5g (0.05 mol) of 70% nitric acid in 15 minutes at 23 - 26°C with stirring. The reaction mixture was stirred for an hour at the same temperature and then extracted with chloroform. The chloroform layer was washed with water, dried over magnesium sulfate and concentrated to obtain 1,1,1-trichloro-2-nitroethane. Yield : 5.8g (65.0%).

Example 6.

5.6g (0.055 mol) of 36% hydrochloric acid and 3.35g (0.05 mol) of 94% fuming nitric acid (d. 1.50) were mixed, to which 4.85g (0.05 mol) of 1,1-dichloro ethylene was dropwise added with in 15 minutes at 23 - 26°C with stirring. The reaction mixture was stirred for an hour at the same temperature and extracted with chloroform. The chloroform layer was washed with water, dried over magnesium sulfate and concentrated to obtain 1,1,1-trichloro-2-nitroethane. Yield : 6.02g (67.5%).

Example 7.

To the mixture of 3.21g (0.055 mol) of sodium chloride and 4.85g (0.05 mol) of 1,1-dichloroethylene were dropwise added 9.45g (0.105 mol) of 70% nitric acid in 15 minutes at 23 - 26°C with stirring. The reaction mixture was stirred at the same temperature for 2 hours and then extracted with ethyl acetate. The extract was washed with water, dried over magnesium sulfate and concentrated to obtain 1,1,1-trichloro-2-nitroethane. Yield : 4.3g (48.2%).

Example 8.

A solution of 9.7g (0.1 mol) of 1,1-dichloroethylene in 10 ml of toluene was dropwise added to the mixture of 11.2g (0.11 mol) of 36% hydrochloric acid and 9.0g (0.1 mol) of 70% nitric acid at 23 - 26°C with stirring. This reaction mixture was stirred for 15 hours at the same temperature. The separated toluene layer was washed with water, dried over magnesium sulfate and concentrated to obtain 1,1,1-trichloro-2-nitroethane. Yield : 12.4g (69.5%).

Example 9.

To a solution of 2.83g (0.0158 mol) of 1,1,1-trichloro-2-nitroethane in 40ml of acetonitrile were added 2.86g (0.0207 mol) of $K_2CO_3$, followed by stirring for 30 minutes at room temperature (15 - 20°C). The reaction mixture was cooled to 5 - 7°C, to which a solution of 2.72g (0.0158 mol) of N-(6-chloro-3-pyridyl)methyl-N-ethylamine in 4ml of acetonitrile was dropwise added and stirred for 45 minutes. To the reaction mixture were added 2.2g (0.016 mol) of $K_2CO_3$ and subsequently 1.6g (0.0206 mol) of methylamine (40% methanolic solution) at 18 - 20°C, followed by stirring for an hour at the same temperature. The reaction mixture was filtered, and the filtrate was concentrated. The residue was purified by a silica gel column chromatography (chloroform/ethanol = 85/15) to afford 2.63g (62.7%) of 1-[N-6-chloro-3-pyridyl)-methyl-N-ethyl]amino-1-methylamino-2-nitroethylene as pale yellowish crystals. mp. 83 - 84°C.

Example 10.

A solution of 6.2g of 1,1,1-trichloro-2-nitroethane in 30ml of chloroform was added to 80ml of 2% sodium hydroxide aqueous solution cooled at 0°C under stirring. Immediately, the aqueous layer was separated and extracted with chloroform. The combined chloroform layers were dried over magnesium sulfate and concentrated to yield 4.0g

of 1,1-dichloro-2-nitroethylene (yield : 81.1%). NMR($CDCl_3$)$\delta$ ppm = 7.65(s)

Example 11.

A solution of 2.4g (0.014 mol) of N-(6-chloro-3-pyridyl)methyl-N-ethylamine, 1.4g (0.014 mol) of triethylamine in 5ml of acetonitrile was dropwise added to a solution of 2.0g (0.014 mol) of 1,1-dichloro-2-nitroethylene in 35ml of acetonitrile at 3 - 5°C with stirring. After stirring for 30 minutes, 4.4g (0.057 mol) of methylamine (40% methanol solution) were added to the reaction mixture and stirred for 30 minutes. The reaction mixture was filtered, and the filtrate was concentrated and purified by a silica gel column chromatography (chloroform/ethanol = 7/1) to afford 2.9g (76.5%) of 1-[N-(6-chloro-3-pyridyl)methyl-N-ethyl]amino-1-methylamino-2-nitroethylene as pale yellowish crystals. This substance was identified as the same substance with that obtained in Example 9.

Example 12

To a mixture of 65.83g (0.65 mol) of 36% hydrochloric acid and 58.51g (0.65 mol) of 70% nitric acid was dropwise added 48.96g (0.5 mol) of 1,1-dichloroethylene (purity : 99%) at 17 - 14.5°C, taking an hour and 16 minutes with stirring. After stirring for about 1.5 hours at 20 - 22°C, the lower layer of the reaction mixture was collected and washed with 45ml of water. The mixture to which 150ml of ethyl ether were added was dried over magnesium sulfate and concentrated to obtain 69.81g (78.3%) of 1,1,1-trichloro-2-nitroethane. The product was distilled under reduced pressure to obtain colorless oil of bp. 82.5 - 84°C/24mmHg. NMR($CDCl_3$)$\delta$ ppm : 5.26(s)

Example 13

The method of Example 12 was repeated by using equivalent amounts of 36% hydrochloric acid and 70% nitric acid but varying their molar ratios to 1,1-dichloroethylene, to obtain 1,1,1-trichloro-2-nitroethane. The results are shown in Table 3.

Table 3

| Molar ratio of 36 % HCl and 70% $HNO_3$ | Yield of 1,1,1-trichloro-2-nitroethane (%) |
|---|---|
| 1.0 & 1.0 | 63.0 |
| 1.1 & 1.1 | 70.5 |
| 1.2 & 1.2 | 76.0 |
| 1.3 & 1.3 | 78.3 |
| 1.4 & 1.4 | 76.9 |
| 1.5 & 1.5 | 76.4 |

Example 14

The method of Example 12 was repeated by using 35% hydrochloric acid and 67.5% nitric acid but varying their molar ratios to 1,1-dichloroethylene, to obtain 1,1,1-trichloro-2-nitroethane. The results are shown in Table 4.

Table 4

| Molar ratio of 35% HCl \ Molar ratio of 67.5% $HNO_3$ | 1.0 | 1.1 | 1.2 | 1.3 | 1.5 | 2.0 |
|---|---|---|---|---|---|---|
| 1.0 | 59.9* | 63.6 | 66.4 | 71.8 | --- | --- |
| 1.1 | 59.4 | 67.5 | 70.6 | 74.2 | --- | --- |
| 1.2 | 65.3 | 69.1 | 73.8 | 77.0 | --- | --- |
| 1.3 | 68.1 | 73.4 | 76.1 | 77.7 | --- | --- |
| 1.5 | --- | --- | --- | --- | 75.7 | --- |
| 2.0 | --- | --- | --- | --- | --- | 66.4 |

* Yield (%) of 1,1,1-trichloro-2-nitroethane

As is clear from Tables 3 and 4, it is found that the good yields are achieved in use of 1.2 - 1.5 moles of each of hydrochloric acid and nitric acid, to one mole of the raw material while the use of 1.1 or less moles is significantly lowered in yield.

Example 15

To a mixture of 4.25g (0.05 mol) of sodium nitrate and 4.85g (0.05 mol) of 1,1-dichloroethylene was dropwise added 10.64g (0.105 mol) of 36% hydrochloric acid at 23 - 26°C in 30 minutes, with stirring. After stirring for 3 hours at the same temperature, the lower layer was collected and 30ml of chloroform were added to it. Then the mixture was dried over magnesium sulfate and concentrated to obtain 3.2g (35.9%) 1,1,1-trichloro-2-nitroethane.

Example 16

To a mixture of 67.7g (0.65 mol) of 35% hydrochloric acid and 60.7g (0.65 mol) of 67.5% nitric acid was added 50ml of chloroform. Then 48.9g (0.05 mol) of 1,1-dichloroethylene were dropwise added at 22 - 28.5°C with stirring and continued to stirr for an hour at 22 - 24°C. The separated chloroform layer was washed with water, dried over magnesium sulfate and concentrated to obtain 64.4g (72.2%) of 1,1,1-trichloro-2-nitroethane.

Example 17

To a mixture of 36.46g (0.30 mol) of 30% hydrochloric acid and 37.81g (0.30 mol) of 50% nitric acid was

dropwise added 19.39g (0.20 mol) of 1,1-dichloroethylene at 25°C with stirring. After stirring for 4 hours at 25 - 30°C, the separated organic layer was washed with water, dried over magnesium sulfate and concentrated to obtain 18.6g (52.2%) of 1,1,1-trichloro-2-nitroethane.

Example 18

To a solution of 2.10g (0.0118 mol) of 1,1,1-trichloro-2-nitroethane in 15ml of chloroform was dropwise added a solution of 2.75g (0.026 mol) of sodium carbonate in 15ml of water at 2°C with stirring, followed by addition of 1.57g (0.01 mol) of N-(6-chloro-3-pyridyl)methyl-N-methylamine. After stirring for 30 minutes, 3.79g (0.042 mol) of 50% dimethylamine were dropwise added to the reaction mixture at 2°C and stirred for 30 minutes under ice-cooling and for 30 minutes at room temperature. The separated aqueous layer was extracted with chloroform. The chloroform layer was dried over magnesium sulfate and concentrated. 4ml of ethyl acetate were added to the residue and the mixture was ice-cooled to yield the precipitate of yellow crystals of 1-[N-(6-chloro-3-pyridyl)methyl-N-methyl)amino-1-dimethylamino-2-nitroethylene. Yield : 2.37g (87.5%). mp. 110 - 112°C.

Example 19

The method of Example 18 was repeated by using 40% methylamine instead of dimethylamine, thereby affording 1.98g (77.1%) of 1-[N-6-chloro-3-pyridyl)methyl-N-methyl]amino-1-methylamino-2-nitroethylene as pale yellowish crystals. mp. 103 - 104°C.

Example 20

To a solution of 2.10g (0.0118 mol) of 1,1,1-trichloro-2-nitroethane in 30ml of tetrahydrofuran was dropwise added a solution of 1.2g (0.0118 mol) of triethylamine in 2ml of tetrahydrofuran under cooling to 0°C and with stirring. To the mixture which was stirred for 30 minutes was dropwise added a solution of 0.99g (0.011 mol) of 50% dimethylamine and 2.4g (0.0237 mol) of triethylamine in 5ml of tetrahydrofuran at - 43°C - -38°C. The mixture was stirred for 30 minutes at the same temperature, to which 1.0g (0.007 mol) of 6-chloro-3-pyridylmethylamine was dropwise added. The mixture was allowed to stand until raising to room temperature, stirred for 30 minutes and then filtered. The filtrate was concentrated, and 1,2-dichloroethane was added to the residue. The mixture was again filtered to remove insoluble substance. The filtrate was concentrated and the residue was purified by silica gel column chromatography using acetone to obtain 1.46g (81.0%) of 1-(6-chloro-3-pyridylmethyl)amino-1-dimethylamino-2-nitroethylene as yellow crystals. mp. 124 - 125°C.

Exampls 21

The method of Example 20 was repeated by using 2-chloro-5-thiazolylmethylamine instead of 6-chloro-3-pyridylmethylamine, to obtain 1-(2-chloro-5-thiazolylmethyl)amino-1-dimethylamino-2-nitroethylene as yellow crystals. Yield:76.4% mp. 101- 102 °C

Example 22

(1) To a mixture of 4,840g (46.46 mol) of 35% hydrochloric acid and 4,337g (46.46 mol) of 67.5% hydrochloric acid was dropwise and with stirring added 3,500g of 1,1-dichloroethylene (purity: 99%) at 22 - 30°C, over 2.5 hours. The mixture was continued to stirr for an hour and the separated lower layer was washed with 2.51 of water to obtain 5,097.3g (79.9%) of crude 1,1,1-trichloro-2-nitroethane.

(2) 20.16g of the above crude product were dissolved in 114ml of chloroform and to the resulting solution was added a solution of 26.34g (0.2486 mol) of sodium carbonate in 114ml of water at 2 - 7°C with stirring. Then, 13.5g (0.0791 mol) of N-(6-chloro-3-pyridyl)methyl-N-ethylamine were dropwise added to the mixture at 5 - 6°C and stirred for 40 minutes. Further, 31.59g (0.407 mol) of 40% methylamine were dropwise added to the mixture at 3 - 7°C, followed by stirring for 30 minutes under ice-cooling and 30 minutes at room temperature. The separated aqueous layer was extracted with 48ml of chloroform. The combined chloroform layers were concentrated, and the residue to which 39ml of ethyl acetate were added was ice-cooled to precipitate pale yellowish crystals of 1-[N-(6-chloro-3-pyridyl)methyl-N-ethyl]amino-1-methylamino-2-nitroethylene. Yield: 18.15g (84.9%). mp. 83 - 84°C

Example 23

To a solution of 5.53g (0.031 mol) of 1,1,1-trichloro-2-nitroethane in 38ml of chloroform was dropwise added 4.5g (0.026 mol) of N-(6-chloro-3-pyridyl)methyl-N-ethylamine under ice-cooling and with stirring. After 10 minutes, a solution of 7.23g (0.068 mol) of sodium carbonate in 38ml of water and after further 15 minutes, 8.67g (0.112 mol) of 40% methylamine aqueous solution were dropwise added to the reaction mixture, followed by stirring for 25 minutes under ice-cooling and for 40 minutes at room temperature. By treating the mixture in the same way as in Example 16, 4.42g (61.9%) of pale yellowish crystals were obtained and this product was coincided with that of Example 22.

Example 24

To a solution of 5.53g (0.031 mol) of 1,1,1-trichloro-2-nitroethane in 65ml of acetonitrile was dropwise added a solution of 4.5g (0.026 mol) of N-(6-chloro-3-pyridyl)methyl-N-ethylamine and 5.87g (0.058 mol) of triethylamine in 15ml of acetonitrile under ice-cooling and with stirring. After 20 minutes, 8.67g (0.112 mol) of 40% methylamine aqueous solution were dropwise added to the reaction mixture, followed by stirring for 30 minutes under ice-cooling and for 30 minutes at room temperature. The reaction mixture was concentrated under reduced pressure. To the residue were added 30ml of water and 50ml of chloroform, followed by well-shaking. The collected aqueous layer was extracted with 30ml of chloroform. The combined chloroform layers were concentrated, and the residue was purified by silica gel column chromatography using chloroform : methanol (5:1). The product was crystallized from ethyl acetate to obtain 3.5g (48.9%) of pale yellowish crystals. The crystals were coincided with the product of Example 22.

Example 25

To a solution of 2.05g (0.0193 mol) of sodium carbonate in 20ml of water were added 2.5g (0.0176 mol) of 1,1-dichloro-2-nitroethylene and 25ml of chloroform under ice-cooling and with stirring. To the resulting mixture was dropwise added 2.7g (0.0158 mol) of N-(6-chloro-3-pyridyl)methyl-N-ethylamine at 3 - 5°C, taking 30 minutes. To the mixture which was stirred for 30 minutes was dropwise added 4.8g (0.0618 mol) of 40% methylamine aqueous solution at 6 - 10°C. Then, the mixture was continued to stirred for 10 minutes at the same temperature and for an hour at room temperature. The separated aqueous layer was extracted with chloroform, and the combined chloroform layers were concentrated. The residue to which 3ml of ethyl acetate were added was allowed to stand overnight at room temperature, to precipitate crystals. The crystals as collected by filtration were washed with ethyl acetate to obtain 3.52g (82.2%) of pale yellowish crystals which were identical with the product of Example 22.

Example 26

To a solution of 2.5g (0.0176 mol) of 1,1-dichloro-2-nitroethylene in 25ml of chloroform was dropwise added 2.7g (0.0158 mol) of N-(6-chloro-3-pyridyl)methyl-N-ethylamine under ice-cooling and with stirring. To the mixture which was stirred for an hour was dropwise added a solution of 2.05g (0.0193 mol) of sodium carbonate in 20ml of water. After 30 minutes, 4.8g (0.0618 mol) of 40% methylamine aqueous solution were dropwise added to the mixture at 6 - 10°C, followed by stirring for an hour at room temperature. The separated aqueous layer was extracted with chloroform. The combined chloroform layers were concentrated, and 3ml of ethyl acetate were added to the residue to yield crystals. The crystals collected by filtration were 3.09g (72.2%), which were identical with the product of Example 22.

The compounds shown in the Table 1 were obtained according to the methods described in Example 9 - 11 or Examples 18 - 26.

This invention made it possible to produce 1,1,1-trichloro-2-nitroethanes at high yield by using the starting materials, which are less expensive and easy to handle, and to have solved all the existing problems. This invention provides a safety and low cost processes for the production of useful insecticidal α-unsaturated-amines in the agricultural field.

## Claims

1. A process for the production of 1,1,1-trichloro-2-nitroethane which comprises reacting 1,1-dichloroethylene of the formula (I):

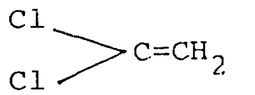

(I)

with nitric acid or its salt and hydrogen chloride or its salt to obtain a compound of the formula (II):

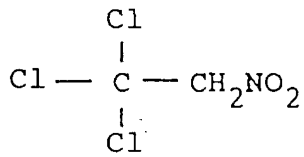

(II),

the reaction being carried out in an aqueous system to which an inert organic solvent may be added, at 0-100 °C in a sealed vessel or at 0-40 °C in an open system, and each of nitric acid and hydrogen chloride being used in 0.5 to 5 equivalents to the compound (I).

**2.** A process for the production of α-unsaturated amines or their salts which comprises reacting 1,1-dichloroethylene of the formula (I) mentioned in claim 1 with nitric acid or its salt and hydrogen chloride or its salt, the reaction being carried out in an aqueous system to which an inert organic solvent may be added, at 0-100 °C in a sealed vessel or at 0-40 °C in an open system, and each of nitric acid and hydrogen chloride being used in 0.5 to 5 equivalents to the compound (I); and subsequently reacting the resulting 1,1,1-trichloro-2-nitroethane of the formula (II) mentioned in claim 1 with an amino compound of the formula (III):

$$R^1\text{-NH-}C_nH_{2n}\text{-A} \qquad \text{(III)}$$

wherein $R^1$ is a hydrogen atom, a $C_{1-4}$alkyl, halo-$C_{1-4}$alkyl, mono- or di-$C_{1-4}$alkoxy-$C_{1-4}$alkyl, $C_{7-9}$aralkyl, optionally substituted phenyl, mono- or di-$C_{1-4}$alkylamino or $C_{1-4}$alkoxy group, A is a 3- or 4-pyridyl, pyrazinyl, 2-, 4- or 5-thiazolyl or phenyl group which may be substituted by a halogen atom, a $C_{1-4}$alkyl, $C_{1-4}$alkylthio or $C_{1-4}$alkoxy group, and n is 0, 1 or 2, or its salt and an amino compound of the formula (IV):

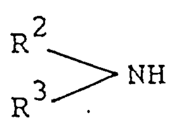

(IV)

wherein $R_2$ is a hydrogen atom, a $C_{1-4}$alkyl or $C_{7-9}$aralkyl group, $R_3$ is a hydrogen atom, a $C_{1-5}$alkyl, halo-$C_{1-4}$alkyl, hydroxy-$C_{1-4}$alkyl, mono- or di-$C_{1-4}$alkoxy-$C_{1-4}$alkyl, mono- or di-$C_{1-4}$alkylthio-$C_{1-4}$alkyl, di-$C_{1-4}$alkylamino-$C_{1-4}$alkyl, tri-$C_{1-4}$alkylsilyl-$C_{1-4}$alkyl, $C_{2-4}$alkenyl, $C_{7-9}$aralkyl, optionally substituted phenyl, amino, di-$C_{1-4}$alkylamino, pyridyl-$C_{1-2}$alkyl or thiazolyl-$C_{1-2}$alkyl group wherein pyridyl and thiazolyl ring may be substituted by a halogen atom, or $R^2$ and $R^3$ together with the adjacent nitrogen atom may form a 5 or 6-membered heterocyclic ring which may contain an oxygen atom or another nitrogen atom or its salt, to obtain a compound of the formula (V):

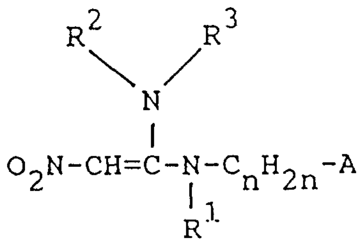

(V)

wherein $R^1$, $R^2$, $R^3$, A and n have the same meanings as defined above or its salt.

3. A process for the prodcution of α-unsaturated amines or their salts which comprises reacting 1,1-dichloroethylene of the formula (I) mentioned in claim 1 with nitric acid or its salt and hydrogen chloride or its salt, the reaction being carried out in an aqueous system to which an inert organic solvent may be added, at 0-100 °C in a sealed vessel or at 0-40 °C in an open system, and each of nitric acid and hydrogen chloride being used in 0.5 to 5 equivalents to the compound (I); and
subsequently reacting the resulting 1,1,1-trichloro-2-nitroethane of the formula (II) mentioned in claim 1 with a base to obtain 1,1-dichloro-2-nitroethylene of the formula (VI)

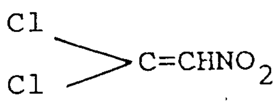

(VI),

and then reacting the 1,1-dichloro-2-nitroethylene (VI) with an amino compound of the formula (III) mentioned in claim 2 or its salt and an amino compound of the formula (IV) mentioned in claim 2 or its salt to obtain a compound of the formula (V) mentioned in claim 2 or its salt.

4. A process claimed in any of claims 1-3 in which the nitric acid is used as 60-70 % nitric acid and its used amount is 1.2 to 1.5 equivalents to the compound (I).

5. A process claimed in any of claims 1-3 in which the compound of the formula (I) is reacted with 60-70 % nitric acid and 25-36 % hydrochloric acid.

6. A process claimed in any of claims 2 or 3 in which when the combination of a primary amine and a secondary amine as the amino compounds of the formulas (III) and (IV) is used, the secondary amine is firstly reacted with a compound of the formula (II) or (VI).

7. A process claimed in any of claims 2 or 3 in which the reaction of a compound of the formula (II) or (VI) with two kinds of amino compounds of the formulas (III) and (IV) is conducted in the presence of a base.

8. A process claimed in any of claims 2 or 3 in which in the formulas (III), (IV) and (V), $R^1$ is a hydrogen atom, a $C_{1-4}$-alkyl group, a halo-$C_{1-4}$alkyl group or a mono- or di-$C_{1-4}$alkoxyalkyl group; $R^2$ is a hydrogen atom or a $C_{1-4}$alkyl group; $R^3$ is a hydrogen atom or a $C_{1-4}$alkyl group; and n is 0 or 1.

9. A process claimed in any of claims 2 or 3 which is adopted to prepare 1-[N-(6-chloro-3-pyridyl)methyl-N-ethyl] amino-1-methylamino-2-nitroethylene, 1-[N-(6-chloro-3-pyridyl)methyl-N-methyl]amino-1-dimethylamino-2-nitroethylene or 1-(6-chloro-3-pyridylmethyl)amino-1-dimethylamino-2-nitroethylene.

**Patentansprüche**

1. Verfahren zur Herstellung von 1,1,1-Trichlor-2-nitroethan, umfassend das Umsetzen von 1,1-Dichlorethylen der Formel (I)

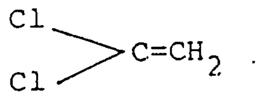 (I)

mit Salpetersäure oder einem Salz davon und Chlorwasserstoff oder einem Salz davon, so dass man eine Verbindung der Formel (II)

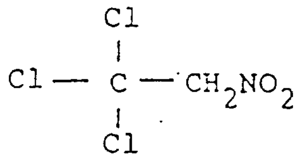 (II)

erhält, wobei die Reaktion in einem wässrigen System, zu dem ein inertes organisches Lösungsmittel gegeben werden kann, bei 0-100 °C in einem verschlossenen Gefäß oder bei 0-40 °C in einem offenen Gefäß durchgeführt wird und die Salpetersäure und der Chlorwasserstoff jeweils in 0,5 bis 5 Äquivalenten in Bezug auf Verbindung (I) verwendet werden.

**2.** Verfahren zur Herstellung von α-ungesättigten Aminen oder ihren Salzen, umfassend das Umsetzen von 1,1-Dichlorethylen der in Anspruch 1 genannten Formel (I) mit Salpetersäure oder einem Salz davon und Chlorwasserstoff oder einem Salz davon, wobei die Reaktion in einem wässrigen System, zu dem ein inertes organisches Lösungsmittel gegeben werden kann, bei 0-100 °C in einem verschlossenen Gefäß oder bei 0-40 °C in einem offenen Gefäß durchgeführt wird und die Salpetersäure und der Chlorwasserstoff jeweils in 0,5 bis 5 Äquivalenten in Bezug auf Verbindung (I) verwendet werden; und
das anschließende Umsetzen des resultierenden 1,1,1-Trichlor-2-nitroethans der in Anspruch 1 genannten Formel (II) mit einer Aminoverbindung der Formel (III)

$$R^1\text{-NH-}C_nH_{2n}\text{-A} \qquad \text{(III)}$$

wobei $R^1$ ein Wasserstoffatom, eine $C_{1-4}$-Alkyl-, Halogen-$C_{1-4}$-alkyl-, Mono- oder Di-$C_{1-4}$-alkoxy-$C_{1-4}$-alkyl-, $C_{7-9}$-Aralkyl-, gegebenenfalls substituierte Phenyl-, Mono- oder Di-$C_{1-4}$-alkylamino- oder $C_{1-4}$-Alkoxygruppe ist, A eine 3- oder 4-Pyridyl-, Pyrazinyl-, 2-, 4- oder 5-Thiazolyl- oder Phenylgruppe, die mit einem Halogenatom, einer $C_{1-4}$-Alkyl-, $C_{1-4}$-Alkylthio- oder $C_{1-4}$-Alkoxygruppe substituiert sein kann, ist und n 0, 1 oder 2 ist, oder einem Salz davon und einer Aminoverbindung der Formel (IV)

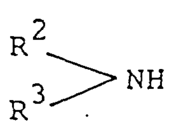 (IV)

wobei $R^2$ ein Wasserstoffatom, eine $C_{1-4}$-Alkyl- oder $C_{7-9}$-Aralkylgruppe ist, $R^3$ ein Wasserstoffatom, eine $C_{1-5}$-Alkyl-, Halogen-$C_{1-4}$-alkyl-, Hydroxy-$C_{1-4}$-alkyl, Mono- oder Di-$C_{1-4}$-alkoxy-$C_{1-4}$-alkyl-, Mono- oder Di-$C_{1-4}$-alkylthio-$C_{1-4}$-alkyl-, Di-$C_{1-4}$-alkylamino-$C_{1-4}$-alkyl-, Tri-$C_{1-4}$-alkylsilyl-$C_{1-4}$-alkyl-, $C_{2-4}$-Alkenyl-, $C_{7-9}$-Aralkyl-, gegebenenfalls substituierte Phenyl-, Amino-, Di-$C_{1-4}$-alkylamino-, Pyridyl-$C_{1-2}$-alkyl- oder Thiazolyl-$C_{1-2}$-alkylgruppe ist, wobei der Pyridyl- und Thiazolylring mit einem Halogenatom substituiert sein kann, oder $R^2$ und $R^3$ zusammen mit dem benachbarten Stickstoffatom einen fünf- oder sechsgliedrigen heterocyclischen Ring bilden können, der ein Sauerstoffatom oder ein weiteres Stickstoffatom enthalten kann, oder einem Salz davon unter Bildung einer Verbindung der Formel (V)

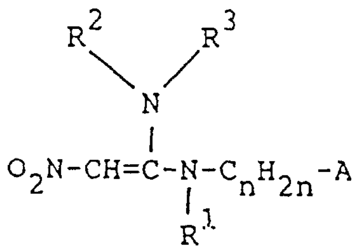

(V)

wobei $R^1$, $R^2$, $R^3$, A und n dasselbe wie oben bedeuten, oder eines Salzes davon.

**3.** Verfahren zur Herstellung von α-ungesättigten Aminen oder ihren Salzen, umfassend das Umsetzen von 1,1-Dichlorethylen der in Anspruch 1 genannten Formel (I) mit Salpetersäure oder einem Salz davon und Chlorwasserstoff oder einem Salz davon, wobei die Reaktion in einem wässrigen System, zu dem ein inertes organisches Lösungsmittel gegeben werden kann, bei 0-100 °C in einem verschlossenen Gefäß oder bei 0-40 °C in einem offenen Gefäß durchgeführt wird und die Salpetersäure und der Chlorwasserstoff jeweils in 0,5 bis 5 Äquivalenten in Bezug auf Verbindung (I) verwendet werden; und
das anschließende Umsetzen des resultierenden 1,1,1-Trichlor-2-nitroethans der in Anspruch 1 genannten Formel (II) mit einer Base unter Bildung von 1,1-Dichlor-2-nitroethylen der Formel (VI)

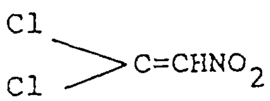

(VI)

und dann das Umsetzen des 1,1-Dichlor-2-nitroethylens (VI) mit einer Aminoverbindung der in Anspruch 2 genannten Formel (III) oder einem Salz davon und einer Aminoverbindung der in Anspruch 2 genannten Formel (IV) oder einem Salz davon unter Bildung einer Verbindung der in Anspruch 2 genannten Formel (V) oder einem Salz davon.

**4.** Verfahren gemäß einem der Ansprüche 1-3, wobei die Salpetersäure als 60-70%ige Salpetersäure verwendet wird und ihre verwendete Menge 1,2 bis 1,5 Äquivalente in Bezug auf Verbindung (I) beträgt.

**5.** Verfahren gemäß einem der Ansprüche 1-3, wobei die Verbindung der Formel (I) mit 60-70%iger Salpetersäure und 25-36%iger Salzsäure umgesetzt wird.

**6.** Verfahren gemäß einem der Ansprüche 2 oder 3, wobei, wenn die Kombination aus einem primären Amin und einem sekundären Amin als Aminoverbindungen der Formeln (III) und (IV) verwendet wird, das sekundäre Amin zuerst mit einer Verbindung der Formel (II) oder (VI) umgesetzt wird.

**7.** Verfahren gemäß einem der Ansprüche 2 oder 3, wobei die Reaktion einer Verbindung der Formel (II) oder (VI) mit zwei Arten von Aminoverbindungen der Formeln (III) und (IV) in Gegenwart einer Base durchgeführt wird.

**8.** Verfahren gemäß einem der Ansprüche 2 oder 3, wobei in den Formeln (III), (IV) und (V) $R^1$ ein Wasserstoffatom, eine $C_{1-4}$-Alkylgruppe, eine Halogen-$C_{1-4}$-alkylgruppe oder eine Mono- oder Di-$C_{1-4}$-alkoxyalkylgruppe ist, $R^2$ ein Wasserstoffatom oder eine $C_{1-4}$-Alkylgruppe ist, $R^3$ ein Wasserstoffatom oder eine $C_{1-4}$-Alkylgruppe ist und n 0 oder 1 ist.

**9.** Verfahren gemäß einem der Ansprüche 2 oder 3, das verwendet wird, um 1-[N-(6-Chlor-3-pyridyl)methyl-N-ethyl] amino-1-methylamino-2-nitroethylen, 1-[N-(6-Chlor-3-pyridyl)methyl-N-methyl]amino-1-dimethylamino-2-nitroethylen oder 1-(6-Chlor-3-pyridylmethyl)amino-1-dimethylamino-2-nitroethylen herzustellen.

## Revendications

**1.** Procédé pour produire du 1,1,1-trichloro-2-nitroéthane, qui comprend la réaction du 1,1-dichloroéthylène de formule (I):

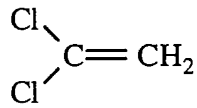

(I)

avec de l'acide nitrique ou un de ses sels, et du chlorure d'hydrogène ou un de ses sels, pour obtenir un composé de formule (II) :

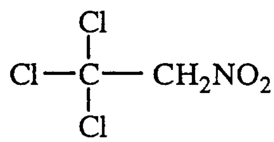

(II)

la réaction étant effectuée dans un système aqueux auquel on peut ajouter un solvant organique inerte, à une température de 0 à 100 °C dans un récipient hermétiquement fermé, ou à une température de 0 à 40 °C dans un système ouvert, et l'acide nitrique et le chlorure d'hydrogène étant utilisés chacun en une quantité de 0,5 à 5 équivalents par rapport au composé (I).

**2.** Procédé pour produire des amines α-insaturées ou leurs sels, qui comprend la réaction du 1,1-dichloroéthylène de formule (I) mentionnée dans la revendication 1, avec de l'acide nitrique ou un de ses sels, et du chlorure d'hydrogène ou un de ses sels, la réaction étant effectuée dans un système aqueux auquel on peut ajouter un solvant organique inerte, à une température de 0 à 100 °C dans un récipient hermétiquement fermé, ou à une température de 0 à 40 °C dans un système ouvert, et l'acide nitrique et le chlorure d'hydrogène étant utilisés chacun en une quantité de 0,5 à 5 équivalents par rapport au composé (I) ; et

la réaction ultérieure du 1,1,1-trichloro-2-nitroéthane résultant de formule (II) mentionnée dans la revendication 1, avec un composé aminé de formule (III) :

$$R^1\text{-NH-}C_nH_{2n}\text{-A} \qquad \text{(III)}$$

dans laquelle $R^1$ représente un atome d'hydrogène, un groupe alkyle en $C_{1-4}$, halogénoalkyle en $C_{1-4}$, mono ou di(alcoxy en $C_{1-4}$)alkyle en $C_{1-4}$, aralkyle en $C_{7-9}$, phényle éventuellement substitué, mono ou di(alkyl en $C_{1-4}$) amino ou alcoxy en $C_{1-4}$ ; A représente un groupe 3- ou 4- pyridyle, pyrazinyle, 2-, 4- ou 5-thiazolyle ou phényle qui peut être substitué par un atome d'halogène, un groupe alkyle en $C_{1-4}$, alkylthio en $C_{1-4}$ ou alcoxy en $C_{1-4}$ ; et n vaut 0, 1 ou 2 ; ou un de ses sels, et un composé aminé de formule (IV) :

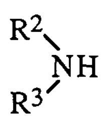

(IV)

dans laquelle $R^2$ représente un atome d'hydrogène, un groupe alkyle en $C_{1-4}$ ou aralkyle en $C_{7-9}$ ; $R^3$ représente un atome d'hydrogène, un groupe alkyle en $C_{1-5}$, halogénoalkyle en $C_{1-4}$, hydroxyalkyle en $C_{1-4}$, mono- ou di (alcoxy en $C_{1-4}$)alkyle en $C_{1-4}$, mono- ou di(alkyl en $C_{1-4}$)thioalkyle en $C_{1-4}$, di(alkyl en $C_{1-4}$)aminoalkyle en $C_{1-4}$, tri(alkyl en $C_{1-4}$)silylalkyle en $C_{1-4}$, alcényle en $C_{2-4}$, aralkyle en $C_{7-9}$, phényle éventuellement substitué, amino, di (alkyl en $C_{1-4}$)amino, pyridyl(alkyle en $C_{1-2}$) ou thiazolyl(alkyle en $C_{1-2}$), les noyaux pyridinyle et thiazolyle pouvant être substitués par un atome d'halogène ; ou $R^2$ et $R^3$ peuvent former ensemble avec l'atome d'azote adjacent un noyau hétérocyclique à 5 ou 6 éléments, qui peut contenir un atome d'oxygène ou un autre atome d'azote ; ou un de ses sels, pour obtenir un composé de formule (V) :

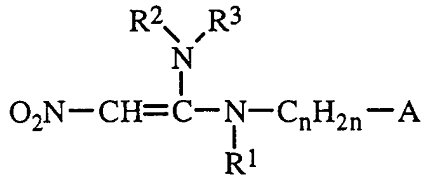

(V)

dans laquelle $R^1$, $R^2$, $R^3$, A et n ont les mêmes significations que celles définies ci-dessus, ou un de ses sels.

**3.** Procédé pour produire des amines $\alpha$-insaturées ou leurs sels, qui comprend la réaction du 1,1-dichloroéthylène de formule (I) mentionnée dans la revendication 1, avec de l'acide nitrique ou un de ses sels, et du chlorure d'hydrogène ou un de ses sels, la réaction étant effectuée dans un système aqueux auquel on peut ajouter un solvant organique inerte, à une température de 0 à 100 °C dans un récipient hermétiquement fermé, ou à une température de 0 à 40 °C dans un système ouvert, et l'acide nitrique et le chlorure d'hydrogène étant utilisés chacun en une quantité de 0,5 à 5 équivalents par rapport au composé (I) ; et

la réaction ultérieure du 1,1,1-trichloro-2-nitroéthane résultant de formule (II) mentionnée dans la revendication 1, avec une base pour obtenir le l,l-dichloro-2-nitroéthylène de formule (VI) :

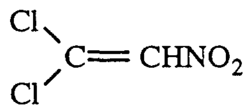

(VI)

puis la réaction du l,l-dichloro-2-nitroéthylène (VI) avec un composé aminé de formule (III) mentionnée dans la revendication 2 ou un de ses sels, et un composé aminé de formule (IV) mentionnée dans la revendication 2 ou un de ses sels, pour obtenir un composé de formule (V) mentionnée dans la revendication 2, ou un de ses sels.

**4.** Procédé selon l'une quelconque des revendications 1 à 3, dans lequel on utilise l'acide nitrique sous la forme d'acide nitrique à 60-70 % et sa quantité utilisée vaut 1,2 à 1,5 équivalents par rapport au composé (I).

**5.** Procédé selon l'une quelconque des revendications 1 à 3, dans lequel on fait réagir le composé de formule (I) avec 60-70 % d'acide nitrique et 25-36 % d'acide chlorhydrique.

**6.** Procédé selon l'une quelconque des revendications 2 et 3, dans lequel, quand on utilise la combinaison d'une amine primaire et d'une amine secondaire comme composés aminés de formules (III) et (IV), on fait tout d'abord réagir l'amine secondaire avec un composé de formule (II) ou (VI).

**7.** Procédé selon l'une quelconque des revendications 2 et 3, dans lequel on mène la réaction d'un composé de formule (II) ou (VI) avec deux sortes de composés aminés de formules (III) et (IV) en présence d'une base.

**8.** Procédé selon l'une quelconque des revendications 2 et 3, dans lequel dans les formules (III), (IV) et (V), $R^1$ représente un atome d'hydrogène, un groupe alkyle en $C_{1-4}$, un groupe halogénoalkyle en $C_{1-4}$, ou un groupe mono- ou di(alkyl en $C_{1-4}$)oxyalkyle ; $R^2$ représente un atome d'hydrogène ou un groupe alkyle en $C_{1-4}$ ; $R^3$ représente un atome d'hydrogène ou un groupe alkyle en $C_{1-4}$ ; n vaut 0 ou 1.

**9.** Procédé selon l'une quelconque des revendications 2 et 3, qui est adopté pour préparer le 1-[N-(6-chloro-3-pyridyl)méthyl-N-éthyl]amino-1-méthylamino-2-nitroéthylène, le 1-[N-(6-chloro-3-pyridyl)méthyl-N-méthyl]amino-1-diméthylamino-2-nitroéthylène ou le 1-(6-chloro-3-pyridylméthyl)amino-1-diméthylamino-2-nitroéthylène.